# EUROPEAN PATENT APPLICATION

(11) **EP 1 529 846 A1**
(43) Date of publication of application: **11.05.2005**
(21) Application number: 03078502.6
(22) Date of filing: 05.11.2003
(51) Int. Cl.: C12Q 1/68

(54) **Means and methods for classifying cyanobacteria**

(71) Applicant: STICHTING VOOR DE TECHNISCHE WETENSCHAPPEN, 3527 JP Utrecht (NL)
(72) Inventor: Kardinaal, Willem Edwin Adrianus, 1091 VT Amsterdam (NL); Janse, Ingmar, 3582 TT Utrecht (NL); Zwart, Gabriel Jacobus Maria, 3607 GA Maarsen (NL)
(74) Representative: Prins, Adrianus Willem, Mr. Ir.

(57) **Abstract**

The present invention is concerned with the typing of cyanobacteria. Methods and nucleic acids for use therein are presented with which preparations comprising a cyanobacterium can be tested and the cyanobacterium therein classified. The classification can be used to correlate the cyanobacterium in the preparation to a pheno- and genotypic characteristics known for the typed strain. This information is useful in the variety of different applications, for instance in predicting and/or characterising toxic blooms of cyanobacteria in surface water bodies.

SEQUENCE LISTING

<110> Stichting voor de Technische Wetenschappen

<120> Means and methods for classifying cyanobacteria

<130> P66834EP00

<140> 03078502.6
<141> 2003-11-05

<160> 46

<170> PatentIn Ver. 2.1

<210> 1
<211> 32
<212> DNA
<213> Artificial Sequence

<220>
<223> Description of Artificial Sequence: primer CYA
      371F

<220>
<221> misc_feature
<222> (1)..(32)

<400> 1
cctacgggag gcagcagtgg ggaattttcc ac                                32

<210> 2
<211> 20
<212> DNA
<213> Artificial Sequence

<220>
<223> Description of Artificial Sequence: primer GC-CSIF

<220>
<221> misc_feature
<222> (1)..(20)

<400> 2
gycacgcccg aagtcrttac                                              20

<210> 3
<211> 18
<212> DNA
<213> Artificial Sequence

<220>
<223> Description of Artificial Sequence: primer 373R

<220>
<221> misc_feature
<222> (1)..(18)

<400> 3

ctaaccacct gagctaat                                                    18

<210> 4
<211> 20
<212> DNA
<213> Artificial Sequence

<220>
<223> Description of Artificial Sequence: primer ULR

<220>
<221> misc_feature
<222> (1)..(20)

<400> 4
cctctgtgtg cctaggtatc                                                  20

<210> 5
<211> 30
<212> DNA
<213> Artificial Sequence

<220>
<223> Description of Artificial Sequence: primer
      (GC-)CYA371F

<220>
<221> misc_feature
<222> (1)..(30)

<400> 5
cctacgggag gcagcagtgg ggaattttcc                                       30

<210> 6
<211> 23
<212> DNA
<213> Artificial Sequence

<220>
<223> Description of Artificial Sequence: primer CYA783R

<220>
<221> misc_feature
<222> (1)..(23)

<400> 6
gactacwggg gtatctaatc ccw                                              23

<210> 7
<211> 19
<212> DNA
<213> Artificial Sequence

<220>
<223> Description of Artificial Sequence: primer ITS3R

<220>

<221> misc_feature
<222> (1)..(19)

<400> 7
tatgcagttt tcaaggttc                                                    19


<210> 8
<211> 40
<212> DNA
<213> Artificial Sequence

<220>
<223> Description of Artificial Sequence: a
      40-nucleotide GC-clamp

<220>
<221> misc_feature
<222> (1)..(40)

<400> 8
cgcccgccgc gccccgcgcc cggcccgccg cccccgcccc                             40


<210> 9
<211> 28
<212> DNA
<213> Artificial Sequence

<220>
<223> Description of Artificial Sequence: probe
      P1a251-52

<220>
<221> misc_feature
<222> (1)..(28)

<400> 9
agcattctct cttatatata gataagat                                          28


<210> 10
<211> 23
<212> DNA
<213> Artificial Sequence

<220>
<223> Description of Artificial Sequence: probe P5b17-52

<220>
<221> misc_feature
<222> (1)..(23)

<400> 10
ctactttttt tcgtctccta cct                                               23


<210> 11
<211> 22
<212> DNA
<213> Artificial Sequence

<220>
<223> Description of Artificial Sequence: probe
      P7-227-52

<220>
<221> misc_feature
<222> (1)..(22)

<400> 11
attcctcgct tcttttcttt ct                                                22


<210> 12
<211> 17
<212> DNA
<213> Artificial Sequence

<220>
<223> Description of Artificial Sequence: probe P5f73-52

<220>
<221> misc_feature
<222> (1)..(17)

<400> 12
agccaatctt gcctcgc                                                      17


<210> 13
<211> 19
<212> DNA
<213> Artificial Sequence

<220>
<223> Description of Artificial Sequence: probe
      P5fmm73-52

<220>
<221> misc_feature
<222> (1)..(19)

<400> 13
aaagccaatt ttgcctcgc                                                    19


<210> 14
<211> 20
<212> DNA
<213> Artificial Sequence

<220>
<223> Description of Artificial Sequence: probe
      P8-245-52

<220>
<221> misc_feature
<222> (1)..(20)

<400> 14
gtaagagcag gtgctgattc                                                   20

<210> 15
<211> 21
<212> DNA
<213> Artificial Sequence

<220>
<223> Description of Artificial Sequence: probe
      P8-221-52

<220>
<221> misc_feature
<222> (1)..(21)

<400> 15
gcttcttttc ttggttttgg c                                                21


<210> 16
<211> 20
<212> DNA
<213> Artificial Sequence

<220>
<223> Description of Artificial Sequence: probe
      P5f-10-52

<220>
<221> misc_feature
<222> (1)..(20)

<400> 16
tcgtcccata cccgaattag                                                  20


<210> 17
<211> 22
<212> DNA
<213> Artificial Sequence

<220>
<223> Description of Artificial Sequence: probe
      P5fmm10-52

<220>
<221> misc_feature
<222> (1)..(22)

<400> 17
tttcgtccca tacctgaatt ag                                               22


<210> 18
<211> 20
<212> DNA
<213> Artificial Sequence

<220>
<223> Description of Artificial Sequence: probe
      P5c213-52

<220>
<221> misc_feature
<222> (1)..(20)

<400> 18
tttctttctt ccacctgtgc                                                    20


<210> 19
<211> 22
<212> DNA
<213> Artificial Sequence

<220>
<223> Description of Artificial Sequence: probe
      P5cmm212-52

<220>
<221> misc_feature
<222> (1)..(22)

<400> 19
cttttctttt ttccacctgt gc                                                 22


<210> 20
<211> 22
<212> DNA
<213> Artificial Sequence

<220>
<223> Description of Artificial Sequence: probe
      P7-221-52

<220>
<221> misc_feature
<222> (1)..(22)

<400> 20
cgcttctttt ctttcttttt gc                                                 22


<210> 21
<211> 22
<212> DNA
<213> Artificial Sequence

<220>
<223> Description of Artificial Sequence: probe
      P7-221mm-52

<220>
<221> misc_feature
<222> (1)..(22)

<400> 21
cgcttctttt ctttgttttt gc                                                 22


<210> 22

<211> 24
<212> DNA
<213> Artificial Sequence

<220>
<223> Description of Artificial Sequence: probe
      P9-246-52

<220>
<221> misc_feature
<222> (1)..(24)

<400> 22
actatgtagg taagataagg tgct                                              24


<210> 23
<211> 25
<212> DNA
<213> Artificial Sequence

<220>
<223> Description of Artificial Sequence: probe
      P9mm242-52

<220>
<221> misc_feature
<222> (1)..(25)

<400> 23
actatgtaag taagataagg tgctg                                             25


<210> 24
<211> 22
<212> DNA
<213> Artificial Sequence

<220>
<223> Description of Artificial Sequence: probe
      Plab30-52

<220>
<221> misc_feature
<222> (1)..(22)

<400> 24
cttggtttcg actactcttt tt                                                22


<210> 25
<211> 24
<212> DNA
<213> Artificial Sequence

<220>
<223> Description of Artificial Sequence: probe
      Plab28mm-52

<220>
<221> misc_feature

<222> (1)..(24)

<400> 25
cttggtttcg actactattt tttc                                              24


<210> 26
<211> 23
<212> DNA
<213> Artificial Sequence

<220>
<223> Description of Artificial Sequence: probe
      P1all36-52

<220>
<221> misc_feature
<222> (1)..(23)

<400> 26
attgattctt ggtttcgact act                                               23


<210> 27
<211> 28
<212> DNA
<213> Artificial Sequence

<220>
<223> Description of Artificial Sequence: probe
      P2all252-48

<220>
<221> misc_feature
<222> (1)..(28)

<400> 27
cattctctct tatatatgta tataagat                                          28


<210> 28
<211> 26
<212> DNA
<213> Artificial Sequence

<220>
<223> Description of Artificial Sequence: probe
      P2all258-52

<220>
<221> misc_feature
<222> (1)..(26)

<400> 28
ccagcattct ctcttatata tgtata                                            26


<210> 29
<211> 28
<212> DNA
<213> Artificial Sequence

<220>
<223> Description of Artificial Sequence: probe
      P2al1241-52

<220>
<221> misc_feature
<222> (1)..(28)

<400> 29
atatatgtat ataagataag gtgctgat                                          28


<210> 30
<211> 19
<212> DNA
<213> Artificial Sequence

<220>
<223> Description of Artificial Sequence: probe
      P2al170-52

<220>
<221> misc_feature
<222> (1)..(19)

<400> 30
aagcctcata cctcgttcc                                                    19


<210> 31
<211> 19
<212> DNA
<213> Artificial Sequence

<220>
<223> Description of Artificial Sequence: probe
      P2a1lmm70-52

<220>
<221> misc_feature
<222> (1)..(19)

<400> 31
aagccacata cctcgttcc                                                    19


<210> 32
<211> 21
<212> DNA
<213> Artificial Sequence

<220>
<223> Description of Artificial Sequence: probe
      P2b5a6-222-52

<220>
<221> misc_feature
<222> (1)..(21)

<400> 32

attcggagct tctcttgttt t                                                21

<210> 33
<211> 22
<212> DNA
<213> Artificial Sequence

<220>
<223> Description of Artificial Sequence: probe
      P2b5de6-34-52

<220>
<221> misc_feature
<222> (1)..(22)

<400> 33
gattcttggt tgggactact tt                                               22

<210> 34
<211> 23
<212> DNA
<213> Artificial Sequence

<220>
<223> Description of Artificial Sequence: probe
      P2b5de6mm33-52

<220>
<221> misc_feature
<222> (1)..(23)

<400> 34
gattcttggt agggactact ttt                                              23

<210> 35
<211> 25
<212> DNA
<213> Artificial Sequence

<220>
<223> Description of Artificial Sequence: probe
      P8-9-259-52

<220>
<221> misc_feature
<222> (1)..(25)

<400> 35
attctctctt actatgtagg taaga                                            25

<210> 36
<211> 26
<212> DNA
<213> Artificial Sequence

<220>
<223> Description of Artificial Sequence: probe

P8-9mm-252-52

<220>
<221> misc_feature
<222> (1)..(26)

<400> 36
cattctctct tactatgtaa gtaaga                                          26


<210> 37
<211> 24
<212> DNA
<213> Artificial Sequence

<220>
<223> Description of Artificial Sequence: probe
      p10111213-246-52

<220>
<221> misc_feature
<222> (1)..(24)

<400> 37
tactatgtca gtaagataag gtgc                                            24


<210> 38
<211> 25
<212> DNA
<213> Artificial Sequence

<220>
<223> Description of Artificial Sequence: probe
      p1011213mm-244-52

<220>
<221> misc_feature
<222> (1)..(25)

<400> 38
ttactatgta agtaagataa ggtgc                                           25


<210> 39
<211> 25
<212> DNA
<213> Artificial Sequence

<220>
<223> Description of Artificial Sequence: probe
      P11-11-52

<220>
<221> misc_feature
<222> (1)..(25)

<400> 39
   tttttttcgt cttttacctg aatta                                            25

<210> 40
<211> 25
<212> DNA
<213> Artificial Sequence

<220>
<223> Description of Artificial Sequence: probe
      P11mm11-52

<220>
<221> misc_feature
<222> (1)..(25)

<400> 40
   tttttttcgt cttatacctg aatta                                          25


<210> 41
<211> 18
<212> DNA
<213> Artificial Sequence

<220>
<223> Description of Artificial Sequence: probe P1494-48

<220>
<221> misc_feature
<222> (1)..(18)

<400> 41
cggttacctt gttacgac                                                 18


<210> 42
<211> 16
<212> DNA
<213> Artificial Sequence

<220>
<223> Description of Artificial Sequence: probe P1514-52

<220>
<221> misc_feature
<222> (1)..(16)

<400> 42
cagccacacc ttccgg                                                   16


<210> 43
<211> 100
<212> DNA
<213> Artificial Sequence

<220>
<223> Description of Artificial Sequence: consensus
      sequence of ITS sequences from different
      microcystis strains wherein n is unspecified

<220>
<221> misc_feature

<222> (1)..(100)

<400> 43
aagggagacc taattcnggt annagacgaa aaaaaagtag tcnctaccaa gaatcaatcc 60
caaaaggtcg gancgaggna nnnntggctt tcaaactagg 100

<210> 44
<211> 99
<212> DNA
<213> Artificial Sequence

<220>
<223> Description of Artificial Sequence: consensus
      sequence of ITS sequences from different
      microcystis strains wherein n is unspecified

<220>
<221> misc_feature
<222> (1)..(99)

<400> 44
ttctgggttc anacaagacc tgaatcagga acaagggcta ttagctcagg tggttagagc 60
gcacccctga taagggtgag gtccctggtt cgagtccag 99

<210> 45
<211> 90
<212> DNA
<213> Artificial Sequence

<220>
<223> Description of Artificial Sequence: consensus
      sequence of ITS sequences from different
      microcystis strains wherein n is unspecified

<220>
<221> misc_feature
<222> (1)..(90)

<400> 45
gatggcccac ctgcacaggt ggcaaaaaca anagaagcga ggaatcagca ccttatctta 60
cttanatagt aagagagaat gctggctctg 90

<210> 46
<211> 70
<212> DNA
<213> Artificial Sequence

<220>
<223> Description of Artificial Sequence: consensus
      sequence of ITS sequences from different
      microcystis strains wherein n is unspecified

<220>
<221> misc_feature
<222> (1)..(70)

<400> 46
agtanagagt ccagaggaac cttgaaaact gcatagagct aggtgaaaaa gccaaaaaag 60

aagaccgcaa 70

## Description

The present invention relates to the field of cyanobacteria, in particular it relates to the development of means and methods for classifying cyanobacteria.

Worldwide, eutrophic freshwater lakes endure noxious blooms and surface scums caused by cyanobacteria from, for instance, the genus Microcystis. During many blooms, Microcystis cells produce the hepatotoxin microcystin which is toxic to humans and many other eukaryotes (3). Microcystins are cyclic heptapeptides, formed non-ribosomally by peptide- and polyketide-synthetases. More than 70 structural microcystin variants are known to date (12).

Water management authorities need to determine whether and to what extent blooms of cyanobacteria can influence the water quality of their surface waters. Not only for the conversion into drinking water but also to maintain a balanced environment in the body water and to prevent toxic effects on recreational users of the water bodies. Toxin measurements of surface freshwater lakes can provide insight into the actual situation at the moment of sampling, however, these immediate tests are not suited when predictions about future development of the toxin levels are needed such as for a proper assessment of the hazards that Microcystis blooms pose on human health and aquatic ecosystems.

Cyanobacteria have been classified using a number of different methods. Traditionally, cyanobacteria are classified on the basis of morphological characteristics. Recently, hybridisation to 16S rRNA probes has been used for classifying this particular group of bacteria. This technique has the disadvantage that much of the variation within the group of cyanobacteria is not seen. The reason is most likely that the 16S rRNA is not divergent enough between the various strains. This leads to an under estimation of the genetic variety in the population of cyanobacteria and indeed in many cases cyanobacteria having identical 16S rRNA sequences differ in other genetic aspects such as but not limited to toxin production. Considering the interest in toxin production by cyanobacteria, tests have been developed to discriminate cyanobacteria on the absence, presence or kind of genetic information that contributes directly to toxin production. However, also this approach has serious limitations in that although providing an indication on the capability of toxin production, it does not give sufficient insight into strain characteristics. Strains characteristics alone or combined with local variation in the conditions in the freshwater environment can have an effect on the absolute levels of toxin produced and coupled therewith the risk for the environment. For instance, the tendency to be a high or relatively low producer of toxin, as well as the array of toxin variants that can be produced, has been found to vary from strain to strain. In addition, the tendency to form scums on the water surface and thus the contribution to toxicity of a bloom may vary from strain to strain.

The present invention provides an alternative method for the classification of cyanobacteria. In the present invention the Internal Transcribed Spacer region (ITS) between 16S rRNA and 23S rRNA is used as a discriminating tool. It has been found that this region is sufficiently divergent to allow an adequate separation between the various strains of cyanobacteria, whereas at the same time it is not so divergent as to confound strain identification. The regions flanking the ITS in the 16S rRNA gene and the 23S rRNA gene are sufficiently conserved among cyanobacteria to allow amplification of this region for most cyanobacteria. As a group, the ITS regions of cyanobacteria are sufficiently far removed from the group of normal bacteria to prevent detecting bacterial strains in the classification method.

With the present classification it is possible to pinpoint groups of genetically sufficiently similar cyanobacteria such that a prediction of their phenotype is reasonably accurate. Thus for the purpose of the present invention, the term strain does not refer to a single clone of genetically identical cyanobacteria, although this can be the case, but rather to a group of cyanobacteria that is genetically very similar. Thus cyanobacteria that belong to a strain have the same ITS sequence in the region determined. In case of a method using a probe, all cyanobacteria samples that hybridise under stringent conditions are said to be of the same strain. Typically this is the case where the cyanobacteria have no mismatch with the probe. In case of denaturing gradient electrophoresis all cyanobacteria samples having the same migration pattern as the reference cyanobacterial sample are said to belong to the same strain. This is typically the case where over the region of the ITS covered, there is no sequence divergence with the reference cyanobacterial sample. In case the sequence is determined, the sample cyanobacterium is said to be of the same strain as the reference cyanobacterium when the ITS sequence over the range sequenced is identical. The similarity in genotype is sufficiently strong that a strain can be coupled to phenotypic characteristics thereby allowing a reasonable accurate prediction of the phenotype of a cyanobacterium identified by means of a method of the invention.

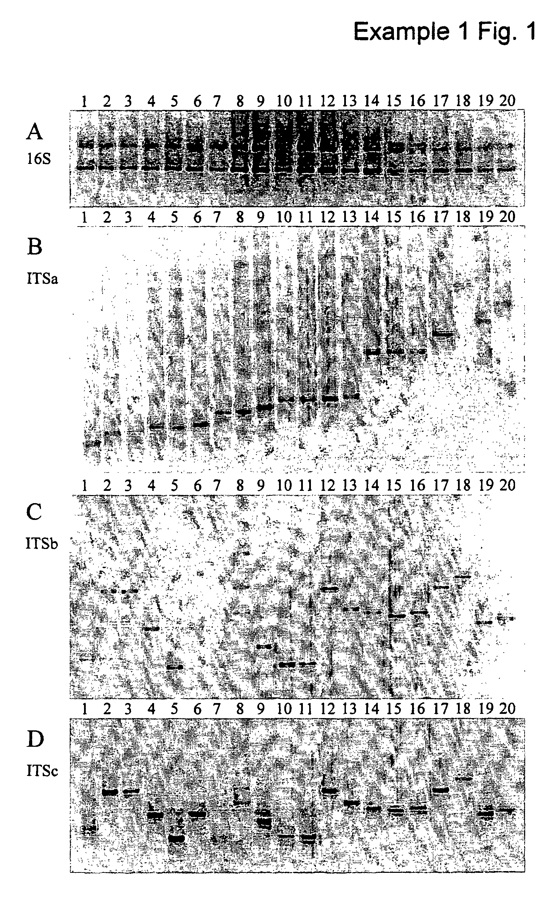

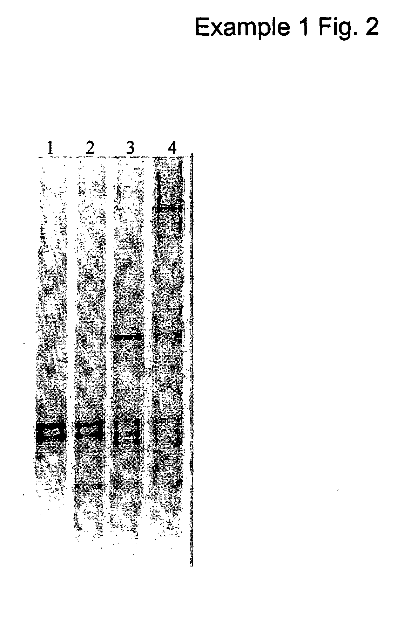

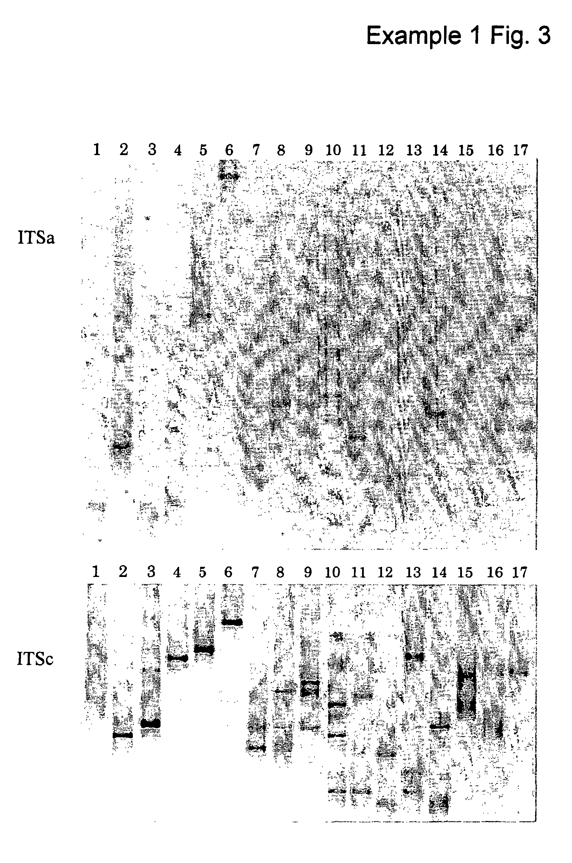

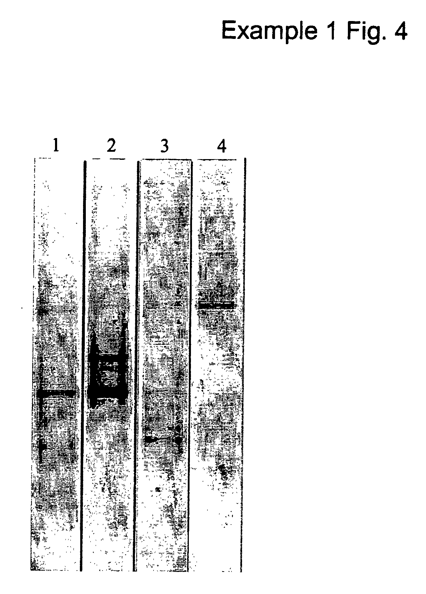

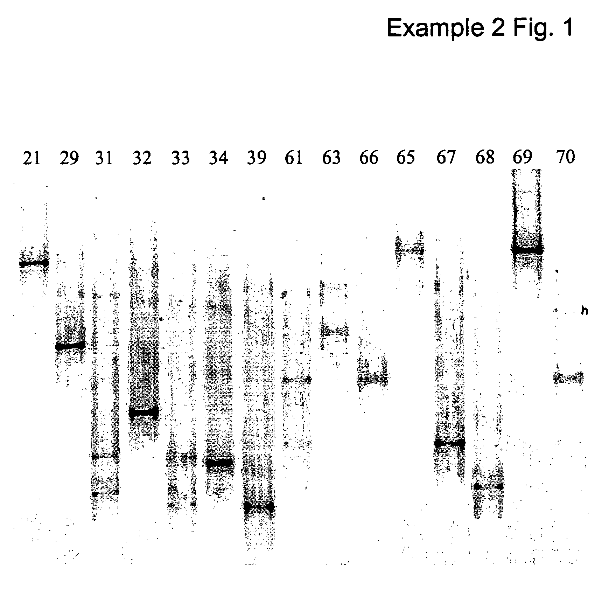

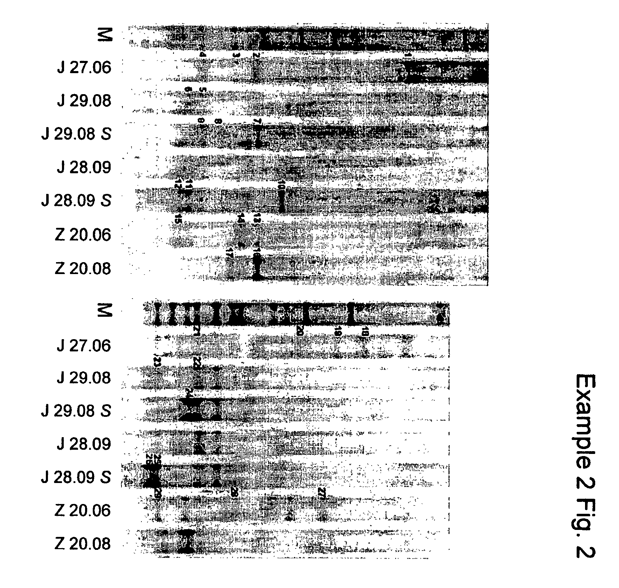

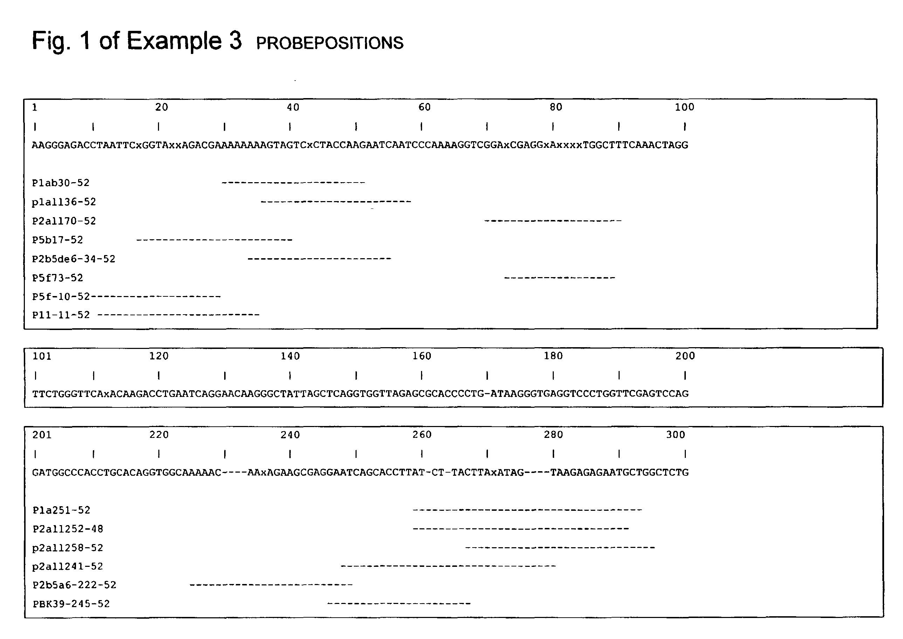

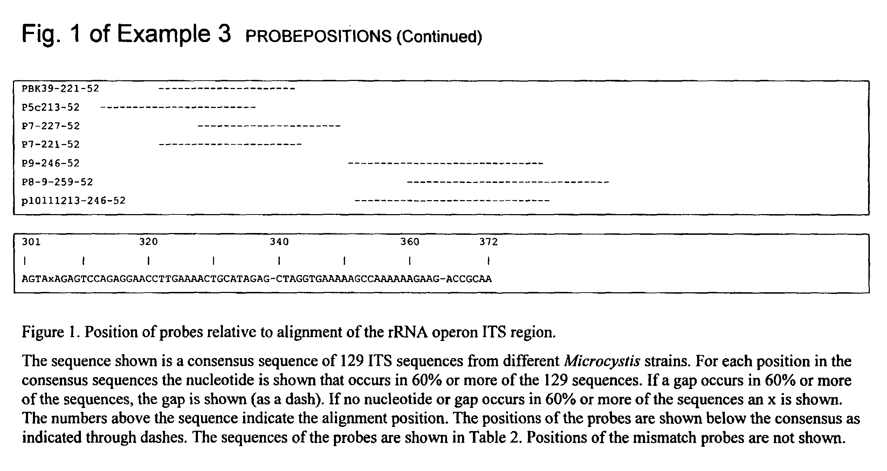

In one aspect the invention provides a method for classifying a cyanobacterium clone comprising providing nucleic acid from said clone with at least one cyanobacteria specific primer, amplifying at least part of the ITS region therein and separating the resulting amplificate under gradually increasing denaturing conditions. This process that in a specific implementation is referred to as DGGE utilizes both the size of the nucleic acid and the sequence content thereof as a discriminating variable. The conditions that initiate denaturation of the nucleic acid vary with the sequence. Some sequences will denature readily whereas other sequences will denature only under significantly stronger denaturing conditions. The migration speed of a nucleic acid through a mesh under the influence of an electrical field is very sensitive to denaturation state of the nucleic acid. Double stranded nucleic acid migrates relatively quickly through the mesh whereas partly denatured nucleic acid migrates very poorly. Thus the migration endpoint under a gradient of ever increasing denaturing conditions is indicative of both the size and of the sequence of the migrated nucleic acid. In the present invention it has been found that this system can favourably be used to discriminate between different cyanobacterium strains. It is particularly suited to discriminate between closely related strains as there are among the genus Microcystis. In a preferred embodiment the cyanobacteria specific primer comprises a sequence as depicted in table 1 of example 3. The primer may comprise a part of a sequence as depicted in table 1 of example 3, however, the primer comprises at least 15 and preferably at least 18 of the nucleotides of the sequences presented in table 1 of example 3. In a preferred embodiment the primer consist of a sequence as depicted in table 1 of example 3. Preferably, a primer comprises one or more of the (GC-)CSIF sequences in table 1 of example 3. The ITS can be subdivided into regions that are of particular interest in the method for the denaturing electrophoresis analysis. In a preferred embodiment the nucleic acid for the electrophoresis comprises the region amplified by primers ((GC-)CSIF and ULR), or by primers ((GC-)CSIF and 373R, or by primers ((GC-)CSIF and ITS3R. Preferably, the primers (GC-)CSIF and ULR are used. Using the primers and/or a method of the invention, cyanobacterial communities and isolates can be studied at high resolution. The selectivity of the primers makes it possible to focus on cyanobacteria in the presence of other organisms. The rationale for using different rRNA-ITS primer sets has become clear from our results. Each set has its advantages and disadvantages, and the choice which one to use depends on the study that is to be executed. For studying cyanobacteria, ITSa primers (GC-)CSIF and 373R have the broadest applicability and produce the most straightforward DGGE profiles. However, bands from some organisms may end up at identical positions in the gel, while sequence differences do exist (either because sequence differences occur in the non-amplified 3'-part of the rRNA-ITS or because different sequences dictate an identical melting behaviour). In case one wants to expand on the information obtained from DGGE of ITSa, for instance to describe an ecosystem in detail or to make sure a culture contains one cyanobacterial isolate one may combine the results obtained with results obtained from longer stretches of rRNA-ITS that are amplified by ITSb primers (GC-)CSIF and ITS3R and ITSc primers (GC-)CSIF and ULR. This makes detection of additional sequence differences possible and thus provides supplementary resolution (Fig. 1 of example 1). Also, these amplicons contain more sequence information, which is especially interesting when field profiles are analyzed. ITSb primers provide more selectivity towards some cyanobacterial genera (Table 3 of example 1), and produce complex DGGE profiles for some genera. Most sequence information, and thus strain typing potential, can be retrieved from ITSc primer amplicons. Moreover, for most tested genera they yielded particularly sharp bands on DGGE gels.

In the present invention it has been found that the ITS region is particularly well suited for the discrimination between different cyanobacterium strains using a probe from that region. It has been found that for many different cyanobacteria it is possible to design probes that discriminates the selected strain from other strains of cyanobacteria. The invention thus provides a method comprising hybridising a probe from an rRNA-internal transcribed spacer (ITS) located between sequences from a 16S and a 23S rRNA gene, of a reference cyanobacterium strain, with a preparation comprising nucleic acid from said region derived from a test cyanobacterium and determining whether hybridisation occurs. The method can be used to determine whether the test cyanobacterium belongs to the strain of the reference cyanobacterium or not.

This information is useful for instance in monitoring a particular cyanobacterium that is present in a lake. For instance, if in past seasons that particular strain caused problems in the lake, one can monitor samples from the lake for the presence of this cyanobacterium.. The method may also be used to determine the relative contribution of the particular strain in the lake. In this embodiment the method further comprises comparing the hybridisation result with a control hybridisation. By using a standard of varying but known amounts of input nucleic acid in the control hybridisation one can compare the hybridisation signal intensity of the sample with the signal intensities in the control hybridisation and determine from that the amount of specific nucleic acid in the test sample. Once this is known one can correlate this to the number of cells and thereby to the concentration of the test cyanobacterium in the sample. If the test cyanobacterium is not the only cyanobacterium in the sample one can also arrive at the relative contribution of the test cyanobacterium to the total population of cyanobacteria in the test sample. Such information can be used for instance to determine the population dynamics of cyanobacteria in a particular body of water. Thus in a further embodiment a method of the invention further comprises classifying said test cyanobacterium on the basis of said hybridisation and/or DGGE analysis. For quantification purposes one can correlate the results obtained with reference samples having various known amounts or distributions of the to be quantified cyanobacterium or cyanobacteria, respectively.

Of current interest is the toxin production capability of at least some strains of cyanobacteria. Thus in a further embodiment the invention provides classifying said test cyanobacterium as a toxin producing strain. It is further possible to further classify toxin of the test cyanobacterium and to relate it to a particular toxin group, and within the group to a certain subtype. For instance the *mcy* gene cluster, encoding the microcystin synthetase genes, exhibits diversity and many different microcystin toxins can be produced by the various strains of Microcystis. With a method of the invention it is possible to correlate the ITS genotype to the toxin genotype. Although there is no direct genetic linkage between the two genotypes, it has been found that horizontal transmission between different cyanobacteria of the two loci is not very frequent. This infrequent horizontal transmission renders the correlation to presence and kind of toxin genes sufficiently predictive for the ITS typed strain. Indeed many more genotypic and also phenotypic characteristics are sufficiently stable to allow correlation with the ITS genotype. Using a method of the invention it is therefore possible to associate further strain pheno- or genotype characteristics to the test cyanobacterium on the proviso of course that such characteristics are determined for the reference cyanobacterium. Considering the close relationship between the various cyanobacteria belonging to a strain as defined in the invention, it is very likely that the classified test cyanobacterium will also exhibit such characteristics. Such characteristics encompass at least, the level of toxin production and if the cyanobacterium produces more than one toxin, the relative ratio of the various toxins produced. Correlations could also be made with growth characteristics such as the colony size, whether or not the cyanobacterium easily forms multicellular structures, floating behaviour, nutritional preferences and/or requirements, and resistance or susceptibility to water quality control measures etc. Thus in another embodiment a classification of the invention further comprises classifying said cyanobacterium as a surface layer forming strain and/or as a high toxin producing strain.
A method of the present invention can be practised using any reference cyanobacterium. However, in a preferred embodiment the reference cyanobacterium comprises a Microcystis cyanobacterium.

With the use of a single probe or reference denaturing gradient gel electrophoresis sample it is possible to determine whether a test cyanobacterium and the reference cyanobacterium belong to the same strain or not. By expanding the number of reference cyanobacteria in the sense that more probes are used and/or that more reference cyanobacterium samples are compared with, it is possible to increase the chance that a particular test cyanobacterium can be typed. Thus in a preferred embodiment the invention provides a method wherein said hybridisation comprises hybridising said preparation with a panel of nucleic acid probes wherein said panel comprises said probe and at least one probe derived from the intergenic region between the transcribed sequences from 16S and 23S rRNA gene of another reference cyanobacterium strain. The probes do not necessarily have to map to the same region of the ITS. In fact many probes are situated in different regions. In a preferred embodiment said panel comprises at least five probes, wherein each of said five probes is derived from the intergenic region between the transcribed sequences from 16S and 23S rRNA gene of different reference cyanobacterium strains. Using five probes is typically sufficient to monitor the population dynamics of cyanobacteria in a particular body of water. Preferably the panel comprises more than ten, and preferably more than 15 of said probes of different cyanobacterium strains. Using these larger number of probes the chance that a test cyanobacterium can assigned to a strain increases. Of course strains that do not belong to one of the strains that a probe is used for (a typed strain), is thereby typed as a new strain and a probe can be designed to identify this new strain, which new probe can subsequently be added to the panel. Upon typing of the strain, the ITS region can be used to further delineate genetic differences and thus further subdivision of the strain. This can for instance be done by comparing the ITS regions on a sequence level.

The probes are typically selected from ITS sub regions wherein at least one nucleotide mismatch is present compared with the sequence of the strain one wants to distinguish from. Typically a probe is selected to have as much discriminating power as possible. This can be achieved by selecting probes having at least one mismatch with all typed cyanobacterial ITS sequences other than those from the strain for which the probe has been designed. Probes having at least one nucleotide mismatch with the closest related counterpart can be used in the present invention. A one nucleotide mismatch is typically sufficient to obtain a clearly distinguishable hybridisation result. A counterpart probe can be designed for the closest related counterpart. This counterpart probe, is completely complementary to the region in the counterpart ITS and has the same number of nucleotides. This counterpart probe can be incorporated into the panel. This allows an even more precise analysis of the hybridisation result. The signal obtained with the probe can be compared with the signal obtained with the counterpart probe. The test cyanobacterium belongs to the same strain as the strain that the probe is derived from, if the hybridisation signal obtained with the probe is above background (i.e. there was specific hybridisation) and simultaneously more intense than the hybridisation signal obtained with counterpart probe. Counterpart probes can be designed for all probes in the panel and likewise be incorporated into the panel. Thus whereas probes can be of different regions in the ITS, counterpart probes always overlap the same region as the probe in the closest related counterpart strain. In a preferred embodiment the probe comprises at least two mismatches with the same region in the closest related counterpart. Thus different clones that have no mismatch within the region of the probe, are typed in the present invention as belonging to the same strain, irrespective of the fact that other geno- or phenotypic differences may be present. Thus in one embodiment of the invention at least one of said probes in the panel comprises no mismatches with the sequence of the reference strain the probe is derived from, and at least two mismatches with the sequence of said region in at least one other reference cyanobacterium strain. Preferably, said at least one other reference strain is the strain that is the closest related counterpart of the reference cyanobacterium strain that the probe is derived from. In a preferred embodiment of the invention, the panel is a dedicated panel, for instance, capable of discriminating between all typed Microcystis strains.
In one embodiment said at least five of said probes comprise no mismatches with the sequence of said region in the reference strains they are derived from, and each of said at least five probes comprises at least two mismatches with the sequence of said region in the other strains of said at least five reference strains. The latter criterion being met when the probe has at least two mismatches with the same region in the closest related strain.
In another embodiment a method of the invention further comprises hybridising a control probe from said rRNA-internal transcribed spacer region (ITS) located between sequences from a 16S rRNA gene and a 23S rRNA gene, with a preparation comprising nucleic acid from said region derived from a test cyanobacterium and determining whether hybridisation occurs, wherein said control probe comprises sequence divergence with some strains of cyanobacteria, but not with other strains of cyanobacteria. In a preferred embodiment said control probe is selective for a cluster of cyanobacteria strains. This probe provides confirmation of the information obtained for the strain specific probe in that there is also hybridisation with the control probe.
It provides further information on the correct strain assignment. Thus preferably said control probe is selective for a cluster of cyanobacteria that comprises the strain that said probe is selective for, preferably said control probe comprises a sequence as depicted in table 3B of example 3. The control probe can also be used separately or in combination with strain specific probes that are not specific for strains of the cluster. In one embodiment a positive signal in the control probe provides information on the cluster the test cyanobacterium belongs to and can therefore be used to correlate cluster specific geno- or phenotype characteristics to the test cyanobacterium.

The probe is preferably an oligonucleotide or a functional equivalent thereof. A functional equivalent of an oligonucleotide comprises the same binding affinity in kind not necessarily in amount as the oligonucleotide itself. Suitable nucleotide mimetics are present in the art such as but not limited to peptide nucleic acid (PNA). Other mimetics include (L)-a-threofuranosyl oligonucleotides (TNA), RNA. In a preferred embodiment at least one of said probes comprises between 12 and 30 nucleotides. In a preferred embodiment at least one of said probes comprises between 16 and 28 nucleotides. The probe may share nucleotide sequence with or be complementary to several distinct regions in the ITS, however, in a preferred embodiment at least between 12 and 30 nucleotides are arranged consecutively in said ITS region. Probes may of course further comprise other sequences, such as for instance for the purpose of incorporating a label that becomes detectable upon hybridisation of the probe to the target nucleic acid. Examples of such probes are the so-called molecular beacon probes. The probes may also comprise nucleotides from one or more other regions of the cyanobacterium genome. Probes may also comprise nucleotide analogues having similar hybridisation characteristics in kind as the nucleotide they are analogous to, though they do not have to have the same characteristics in amount. Probes may further comprise other nucleotide analogous such as for instance inosine, that do not have a strong preferred counterpart base with which they associated in the opposing strand. Both types of analogues may be used to accommodate binding strength of particular probes thereby allowing fine adjustment of the panel. This fine tuning can be used to arrive at standardized hybridisation conditions. Probes may also further comprise other matter such as but not limited to protein, lipids or other substances to provide further functionality. In a panel the probes are typically selected to have about the same Tm. This being the case where the Tm of the various probes differs no more than one degree from each other, however, this need not always be true. One may further fine tune the probes to have an even closer set of Tm values. A probe can further be scrutinized for other features such as internal repeats, low self hybridisation characteristics, low internal hybridisation characteristics an low amounts of direct repeats. These properties can all be fine tuned and increase the propensity with which a suitable panel is designed. Probes may also further comprise organic or inorganic compounds, such as can be used to bind the probes to a solid surface or binding matrix and to provide distance from this solid surface or binding matrix.

A probe of the invention preferably comprises a nucleic acid sequence as depicted in table 3 of example 3 or a functional equivalent thereof. A functional equivalent in this case also comprises the reverse complement of the presented sequence. A panel of probes preferably comprises at least one of probes comprising a nucleic acid sequence as depicted in table 3 of example 3 or a functional equivalent of such an oligonucleotide, preferably at least two, and more preferably at least five probes of said panel comprise a nucleic acid sequence as depicted in table 3 of example 3 or a functional equivalent of such an oligonucleotide.

A method of the invention can be performed using any preparation containing nucleic acid of the ITS region. In a preferred embodiment the preparation comprises nucleic acid that has undergone an amplification step specific for the ITS region. By amplifying the nucleic acid from this region, two things are accomplished simultaneously. On the one hand the amount of nucleic acid to be detected is amplified thereby increasing the sensitivity of the method and allowing detection of cyanobacteria that are not easy to grow or obtain in other ways. On the other hand, the potential for background hybridisation of sequences that are not derived from the ITS region is reduced, thereby further increasing the signal to noise ratio for the assay. So in a preferred embodiment said preparation of nucleic acid of said test cyanobacterium is at least in part prepared through amplification of a sample comprising nucleic acid of said test cyanobacterium. Preferably said amplification is performed using a cyanobacterium specific primer within the 16S, 23S or 16S-23S rRNA internal transcribed spacer gene sequence. In this way, the potential for background hybridisation with nucleic acid from bacterial origin other than cyanobacterial origin is substantially reduced. Moreover, specific primers enhance the sensitivity for detection of cyanobacterial ITS sequences, since there is no competition for amplification of non-cyanobacterial sequences during the PCR reaction. Various cyanobacterial specific ITS amplification primers can be designed. Many amplification methods rely on two opposing primers. It is sufficient that at least one of said primers is specific for cyanobacteria. However, preferably, both primers in strategies relying in two opposing primers are cyanobacteria specific. It is possible to perform so-called nested amplifications to further increase the sensitivity of a method of the invention. Such nested primers can be both cyanobacterial specific primers and primers that recognize a broader range of bacterial ITS regions. Preferably, also at least one nested primer(s) is(are) cyanobacterial specific. In a preferred embodiment a cyanobacterial specific primer comprises a sequence as depicted in table 1 of example 3.

The invention further provides at least one of the sequence as identified by the EMBL accession numbers AJ605140 till AJ605221 (see table 2 of example 3). The EMBL sequences refer to ITS regions of particular strains of cyanobacteria that are used to select the probes from. The ITS regions can further be used to characterise the strain from which it was identified. Thus the present invention further provides a method for typing a strain of cyanobacteria comprising determining whether the test cyanobacterium comprises a sequence of one of the isolates identified by accession numbers AJ605140 till AJ605221 as referred to in table 2 of example 3. This method is preferably performed by sequencing amplified product from an ITS region of the test cyanobacterium, however, other sequencing methods are known in art and can also be used.

In yet another embodiment the invention provides a nucleic acid or a functional equivalent thereof as depicted in table 3 of example 3. Further provided is a panel of nucleic acids, or functional equivalents thereof wherein said panel comprises at least one of said nucleic acids comprise a sequence as depicted in table 3 of example 3. Preferably, said panel comprises at least two and more preferably at least five of said nucleic acids wherein said at least five nucleic acids comprise a sequence as depicted in table 3 of example 3 or a functional equivalent thereof.

The panel preferably comprises an array that is associated with a solid surface. Preferably, a flat solid surface. In a particularly preferred embodiment the panel is present on a chip, which has preferably the dimensions of a microscopic slide. Further provided is an apparatus for analysing hybridisation signals comprising a nucleic acid having a sequence depicted in table 3 of example 3, a panel of nucleic acids according to invention, an array and/or a chip according to the invention. For detection of chemiluminescence signals the apparatus can for instance be an X-ray film, a CCD camera (especially the cooled variant), a reader for microtiterplates capable of detecting chemiluminescence signals. For detection of fluorescence signals, array readers such as the SensiChip DNA Array Reader from Quiagen, the Affymetrix Chip Reader, The Genapta microarray reader and microtiterplate readers such as that from Perkin Elmer, Wallac and Pharmacia. For detection of light absorption microtiterplate readers.

In yet another embodiment the invention provides the use of a nucleic acid or functional equivalent thereof of table 3 of example 3 or of a sequence identified by EMBL accession numbers AJ605140 till AJ605221, an array, chip or apparatus according to the invention, for classifying a cyanobacterium. In a preferred embodiment said classification comprises classifying said cyanobacterium as a toxic or non-toxic cyanobacterium strain.

As mentioned above, the invention further provides a method for typing a cyanobacterium in a sample comprising subjecting ITS sequences from said clone to a denaturing gradient gel electrophoresis step and typing the strain from said step. A method of the invention is preferably performed using a clone of the test cyanobacterium as a source to prepare the preparation or as a source of ITS sequences. This source is particularly preferred in the instances wherein the nucleic acid sequence is determined or wherein a denaturing gradient gel is used. A method comprising a denaturing gradient gel electrophoresis step preferably further comprises comparing the result of said step with a reference. The reference is preferably a similar treated sample of a reference cyanobacterium. Preferably, the reference comprises similar treated samples from at least 5 reference and more preferably at least 15 reference cyanobacteria. Preferably, the material used to generate the references comprises a sequence as depicted in table 2 of example 3. Preferably, ITS sequences are provided through amplification of the ITS region of said cyanobacterium clone. The ITS sequences are preferably amplified by using a cyanobacterium specific primer within the 16S, 23S or 16S-23S rRNA internal transcribed spacer gene sequence, preferably by using a cyanobacterium specific primer comprising a sequence as depicted in table 1 of example 3. The reference(s) are preferably similarly treated.

### Examples

### Example 1

### High Resolution Differentiation of Cyanobacteria by Using rRNA Internal Transcribed Spacer Denaturing Gradient Gel Electrophoresis

For many ecological studies of cyanobacteria, it is essential that closely related species or strains can be discriminated. Since this is often not possible using morphological features, cyanobacteria are frequently studied using DNA based methods. We realized high-resolution discrimination of a variety of cyanobacteria by means of DGGE analysis of the internal transcribed spacer, or parts thereof between the 16S and 23S rRNA genes (rRNA-ITS). A forward primer specific for cyanobacteria was designed, targeted at the 3'-end of the 16S rRNA gene. Combination of this primer with three different reverse primers targeted at the rRNA-ITS or the 23S rRNA gene yielded PCR products of different sizes from cultures of all 16 cyanobacterial genera that were tested, but not from bacteria that were not cyanobacteria. DGGE profiles produced from the shortest section of rRNA-ITS consisted of 1 band for all but one cyanobacterial genera, those generated from longer stretches of rRNA-ITS yielded DGGE profiles containing 1 to 4 bands. The method is suited to detect intrageneric and intraspecific variation of cyanobacteria, the method was exemplified using strains of the genus Microcystis. Many strains could be discriminated by means of rRNA-ITS DGGE, and the resolution of this method was strikingly higher than that obtained with previously described methods. The applicability of the developed DGGE assays for analysis of cyanobacteria in field samples was demonstrated using samples from freshwater lakes. Advantages and disadvantages associated with the use of each developed primerset are discussed.

### INTRODUCTION

Cyanobacteria are a major component of many aquatic and terrestrial ecosystems and can be found in virtually all habitat types, including marine and freshwater environments, deserts, hot springs, hypersaline environments, rocks and ice. In addition, they can be involved in symbiotic associations with a remarkable range of eukaryotic host organisms. In all these environments, cyanobacteria are major contributors to photosynthesis and nitrogen fixation (38). Cyanobacterial populations may reach high densities during blooms in surface waters. Detrimental effects of such blooms are of growing concern for water managers worldwide (9).
For ecological studies of cyanobacteria it is essential to be able to differentiate between closely related organisms. Some important features such as nitrogen fixation or toxicity are not uniformly dispersed across taxa or morphological groups, and may vary between species or even between strains of the same species (9, 41). For example, toxic cyanobacterial blooms often require characterization below the genus level. Both toxin producing and non-producing strains are included in most morphologically distinguishable species of the genera Microcystis (18, 30) and Anabaena (5). Shifts in the ratio of toxic and non-toxic genotypes of Microcystis may (partly) explain the often poor correlations of cell counts with toxin content in many field studies (6).
To study the functions and interactions of cyanobacteria, it is necessary to reveal the composition and dynamics of their populations. Also, it is important to relate isolated strains to their counterparts in nature, for extrapolation of findings from physiological experiments carried out on cultures to natural conditions. Linking laboratory cultures and field populations is often problematic due to the selectivity inherent to cultivation, and to morphological changes occurring after cultivation (27, 31). Denaturant gradient gel electrophoresis (DGGE), a technique for sequence-dependent separation of PCR products (25), can be used to assess the genotypic diversity in environmental samples and to judge the purity and uniqueness of isolated strains. Isolated cultures can be assigned to field populations based on the comparison of their DGGE profiles, and sequence information from profile bands can be used to characterize the organisms that are present. A section of DNA is suitable for DGGE analysis if it can be specifically amplified from the target organisms, has sufficient sequence heterogeneity for the desired resolution and, preferably, is part of a gene of which a considerable number of sequences have been deposited in sequence databases. DGGE of hetR, a gene assigned to heterocyst differentiation, has been used to study the diversity in isolated strains of the cyanobacterial genera Trichodesmium and Nostoc (28, 32). Also nifH, a gene encoding dinitrogenase reductase in many microorganisms including cyanobacteria (42), has been used for DGGE analysis of the very diverse functional group of diazotrophic (N2 -fixing) organisms (22). An important drawback for the application of both protein-encoding genes, especially for the analysis of communities of cyanobacteria, is that they are present only in a limited number of cyanobacterial genera. Nübel et al. (27) developed primers for specific amplification of a 16S rRNA gene segment from cyanobacteria and plastids, allowing DGGE analysis of cyanobacterial populations (1, 11, 27). However, the taxonomic resolution offered by 16S rRNA genes is insufficient for discrimination of closely related organisms. As a result, research has increasingly focused on the rRNA 16S-23S internal transcribed spacer (rRNA-ITS). The greater degree of sequence heterogeneity, as well as a considerable number of published rRNA-ITS sequences, make rRNA-ITS very suitable for high-resolution analysis of cyanobacteria. Restriction enzyme digestion (RFLP) of rRNA-ITS has been used to resolve closely related cyanobacterial strains (21, 23, 26, 32), and direct sequencing has been used to study subgeneric phylogenetic relationships in genera such as Microcystis (30), Trichodesmium (28) and picocyanobacteria (34). Recently, rRNA-ITS sequences have been used for analysis of Synechococcus (4) and Aphanizomenon (20).
In the present work, we combine the relatively high sequence variation in rRNA-ITS with the potential of DGGE to separate even small sequence differences, to introduce methods for high-resolution analysis of cultures and populations of cyanobacteria. We developed and tested primers and protocols for selective amplification and DGGE analysis of cyanobacterial genotypes in the presence of DNA from contaminating microorganisms. Their value for analysis of cyanobacterial strains of various genera and of field samples was examined, and the resolution that could be achieved by different rRNA DGGE methods was compared using closely related Microcystis strains.

### MATERIALS AND METHODS

Sampling and DNA Isolation. Lakes Kinselmeer and Sneekermeer are shallow lakes (maximum depth approximately 3 m), and Volkerak and Zeegerplas are deeper lakes (maximum depth 15m and 25 m respectively). All lakes have extensive blooms of various cyanobacteria, mostly in spring and summer. Water samples from the lakes were collected 0.5 m below the surface in sterile bottles from a boat in the middle of the lake and stored in the dark at 4°C. Within four hours after sampling, a volume of 250 ml water (or less if the filter clogged) was filtered over a 25 mm diameter, 0.2 mm pore size mixed esters filter (ME 24, Schleicher & Schuell, Dassel, Germany). The filter was cut in two with a sterile scalpel and each half was stored in a microcentrifuge tube at -80°C until further processing. DNA from filters containing field samples was isolated as described by Zwart et al. (43). DNA from the pellet of 2 ml cyanobacterial cultures in which growth was visible by eye was isolated by means of the DNA isolation procedure described by Tillett et al. (37).
Strain isolation and culture conditions. Strains of cyanobacteria used in this study are given in Table 1. Cultures isolated in this study (Microcystis sp. and Gloeotrichia sp.) were obtained by picking single colonies from water samples using a dissecting microscope and sterile glass Pasteur pipettes with a narrow opening, followed by repeated washing in a small volume of O2 medium (8). Alternatively, single Microcystis colonies were grown on plates containing O2 medium solidified with agarose (0.3% w/v). Colonies grown on plates were rendered unialgal through repeated plating. None of the cyanobacterial cultures that were used in this study were axenic. All cyanobacteria able to grow in freshwater medium were maintained in our laboratory in 02 medium (5 ml in 30 ml tubes or 30 ml in 100 ml Erlenmeyer flasks) at 20°C, at an irradience of approximately 15 mmol×m-2×s-1 at a light:dark cycle of 16:8 h. A Microcoleus isolate was maintained as decribed by Jonkers et al. (16). The bacteria used as negative controls to test the specificity of the primers were Serratia marcescens (DSM 1636), Acinetobacter calcoaceticus strain BD4 (DSM 586), Erwinia carotovora carotovora (DSM 30168), Escherichia coli (DSM 423), Rhodococcus erythropolis (DSM 43188), Lactobacillus reuteri (DSM 20016), Lactococcus lactis lactis (NIZO-81), Bacillus polymixa (DSM 36), Bacillus subtilis (DSM 10), and Pseudomonas stutzeri (DSM 5190). Cultures were obtained from the German Collection of Microorganisms and Cell Cultures (cultures encoded DSM), and from the Dutch Institute for Dairy Research (encoded NIZO).
Sequence alignment and investigation. Sequences of the 16S rRNA of bacteria and cyanobacteria were obtained from the ribosomal database project (RDPII) (24) and Genbank/EMBL/DDBJ databases. The program Dedicated Comparative Sequence Editor (DCSE) (10) and the program package ARB (www.ARB-home.de) were used for sequence alignments on the basis of comparison of secondary structural elements in the ribosomal RNA. These alignments containing most available cyanobacterial sequences, and an alignment of 218 representative bacterial sequences in RDPII, were used to search for potential primersites in the 16S rRNA gene. The number of mismatches of the designed primer with deposited sequences, was investigated using the probe match option in ARB and the '6spring2001' database supplemented with 19 sequences (W. Ludwig and A. Ernst, personal communication) containing 8229 16S rRNA gene sequences of more than 1400 bp (of which 145 are from cyanobacteria and 77 from chloroplasts). Based on this database, databases were constructed containing either all cyanobacteria , all plastids, or all sequences except cyanobacteria and plastids. The number of sequences with a given number of mismatches was determined using probe match, followed by a correction for degenerate base positions in the primer sequence. Sequences from the rRNA-ITS region were obtained from Genbank/EMBI.JDDBJ and were aligned using the programs ClustalW and the BioEdit Sequence Alignment Editor (12).
PCR amplification. Primer sequences and references are given in Table 2. The forward primer for amplification of part of the 16S rRNA gene was slightly modified from Nubel et al (27) to further improve their theoretical specificity. This because the efficiency of amplification, and thereby the selectivity of the primer, is typically determined by the nature of the 3' end nucleotides. (19, 35). PCR amplification was performed in a MBS 0.5 S thermocycler (ThermoHybaid, Ashford, UK) in a 25 ml reaction mixture containing approximately 50 ng of DNA, [10 mM Tris/HCl pH 8.3, 50 mM KCl, 0.01% w/v gelatin, 200 mM of each deoxynucleotide, 1.5 mM MgCl2, 2.5 units of Taq DNA polymerase (Boehringer Mannheim, Mannheim, Germany) and 0.5 mM of each primer]. The temperature cycling conditions for the amplification of part of the 16S rRNA were modified slightly from (27). After a pre-incubation at 94°C for 5 min, a total of 30 cycles were performed of 94°C for 1 min, 60°C for 1 min and 72°C for 1 min. The temperature cycling was concluded with a final step of 5 min at 72°C. The optimized temperature cycling conditions for the amplification of rRNA-ITS (ITSa, ITSb, and ITSc, for explanation see results section and Table 2) were as follows. After a pre-incubation at 94°C for 5 min, a total of 30 cycles were performed of 94°C for 1 min, Ta for 1 min and 72°C for 1 min. In the first twenty cycles, Ta decreased by 1°C after every second cycle from 62°C in the first cycle to 52°C in the twentieth. This 'touch down' procedure was followed to reduce non-specific annealing of the primers. In the last ten cycles, Ta was 52°C. The temperature cycling was concluded with a final step of 30 min at 72°C.
DGGE profiling. Theoretical melting profiles were constructed using the program MELT94 which can be found on the Internet at http://web.mit.edulosp/www/melt.html. DGGE was performed essentially as described by Muyzer et al. (25). Briefly, PCR products were separated on a 1.5 mm thick, vertical gel containing 8% (w/v) polyacrylamide (37.5:1 acrylamide: bisacrylamide) and a linear gradient of the denaturants urea and formamide, increasing from 25 or 30% at the top of the gel to 40% at the bottom. The concentrations depended on the gene products that were analyzed and are given in the figure legends. Here, 100% denaturant is defined as 7 M urea and 40% v/v formamide. Electrophoresis was performed in a buffer containing 40 mM Tris, 40 mM acetic acid, 1 mM EDTA, pH 7.6 (0.5 x TAE) for 16 hours at 75 V. The gel was stained for 1 h in water containing 0.5 µg ml-1 ethidium bromide. An image of the gel was recorded with a CCD camera system (Imago, B&L Systems, The Netherlands).
Sequencing of DNA from DGGE bands. A small piece of gel from the middle of the target band was excised from the DGGE gel and incubated in 50 ml sterile milli-Q for 24 hours at 4°C. The eluent was reamplified using the original primerset and run on DGGE to confirm its identity. For sequence analysis, the eluent was reamplified with reverse primers that had M13 priming sites added 5' of the original primers. PCR products were purified using the Concert Rapid PCR Purification System (GibcoBRL Life Technologies, UK), and these products were used as templates for sequencing reactions with the Thermo Sequenase Primer cycle sequencing kit (Amersham Pharmacia Biotech, USA). Sequencing reaction products were analyzed on an ALF Express II sequencer (Amersham Pharmacia Biotech, USA) with CY5 fluorescence labeled M13 sequence primers or a labeled GC-clamp. The sequences were deposited at EMBL and were assigned accession numbers AJ579895-AJ579906. Sequences were processed using the program sequencher version 4.0.5 (Gene Codes Corp., MI, USA) and similarity with sequences deposited in Genbank/EMBL/DDBJ was checked using the program Blast (2)(via
http://www.ncbi.nlm.nih.gov/BLAST/).

Template mixtures experiment. ITSa was amplified from the selected cultures, and for each strain the amount of PCR product was estimated from an agarose gel. The PCR products were mixed and diluted in order to obtain template DNA from selected cultures, or from mixtures thereof, all in identical final concentrations. This template DNA was used for ITSa amplification and the PCR products were analyzed on a DGGE gel.

### RESULTS

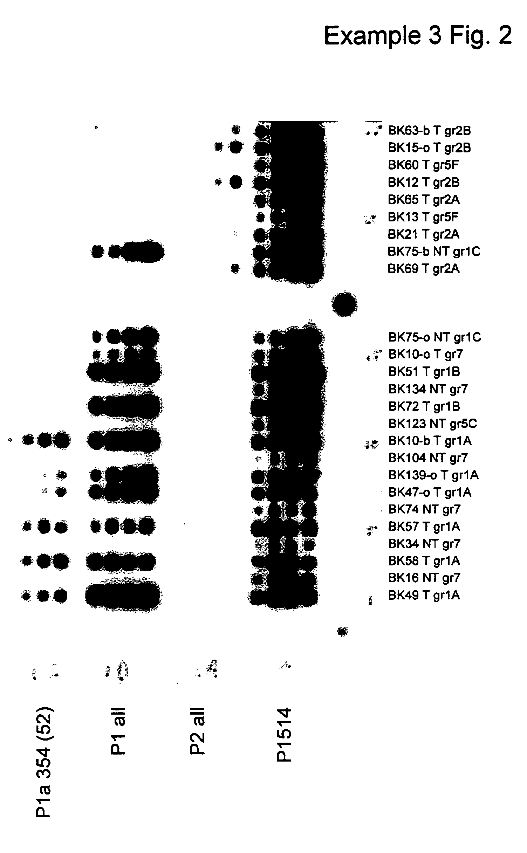

Design of primers for rRNA-ITS amplification. Our aim was to develop a PCR for specific amplification of cyanobacterial rRNA-ITS in the presence of non-cyanobacterial DNA, yielding PCR products that can be separated by means of DGGE. To ensure specific amplification of the rRNA-ITS gene section, the target area for at least one of the primers had to be exclusive for cyanobacteria. For the design of a specific primer we focused on the 16S rRNA gene, since ample sequence information is available from this gene, and it includes areas with various degrees of sequence conservation. We used alignments of all available 16S sequences from cyanobacteria, and of sequences from representative other prokaryotes in the ribosomal database project (RDPII) (24) and Genbank/EMBL/DDBJ databases. The alignments were searched upstream starting from the 3'-end of the 16S rRNA gene, which resulted in the identification of a 20 nucleotide sequence (CSIF, see Table 2) corresponding to position 1423-1442 in the Synechococcus sp. PCC6301 sequence. This sequence was highly conserved in all phylogenetic groups of cyanobacteria from which sequences were available. Out of 145 cyanobacterial sequences, 84 had 0 mismatches, 46 had 1 mismatch, 2 had 2 mismatches, and 13 had 3 mismatches with the primer sequence as revealed by a probe search in ARB. Sequences with three mismatches were found in the genera Nostoc, Spirulina, Gloeobacter and Calothrix. From the first two genera there were also sequences deposited with fewer mismatches. Importantly, the three bases at the 3'-end (TAC), which are the most selective bases when the sequence is used as forward primer (19, 35) appeared to be conserved almost exclusively amongst cyanobacteria. Only the few deposited sequences of the genera Gloeobacter and Calothrix, and a sequence related to Limnothrix redekei misnamed as Oscillatoria limnetica, did not contain a C at the 3'-end. As expected from their close relationship with cyanobacteria, plastid sequences had relatively few mismatches with the primer sequence. A probe search in ARB revealed that from 77 plastid sequences, 1 had a perfect match, 45 had one or two mismatches and 31 had three or more mismatches. To confirm the specificity for cyanobacteria of the primer sequence, a combined probe search was performed in the RDPII database (using the check_probe program) and in an ARB database containing more than 8000 full length 16S rRNA prokaryotic sequences (using the probe match option in ARB). No sequences of prokaryotes other than cyanobacteria were found with less than three mismatches, 2 sequences (from Ammonifex degensii and Thermodesulfobacterium hverag) had 3 mismatches, 116 sequences had 4 mismatches, and the remainder had 5 or more mismatches. A BLAST search (2) of the primer sequence yielded only cyanobacteria in the hits with highest similarity scores.
Reverse primer sites for the amplification of cyanobacterial rRNA-ITS sequences were gathered from previous research (Table 2). Three reverse primers were used, two of these (373R and ITS3R) targeted at highly conserved sequence motifs in the ITS, and one (ULR) at the 5'-end of the 23S gene. The combination of the forward primer (CSIF) and the reverse primers yields three primersets, hereafter denoted as ITSa (primers CSIF + 373R), ITSb (CSIF + ITS3R), and ITSc (CSIF + ULR) (Table 2). The rationale for the use of more than one primer combination for amplification of the rRNA-ITS was the tradeoff that exists between the broad applicability of the shorter fragments and the different melting behavior and higher amount of sequence information associated with longer fragments (see below). Alignment of cyanobacterial rRNA-ITS sequences deposited in the Genbank/EMBL/DDBJ databases showed that virtually all cyanobacteria had at least one operon containing a perfect match with the reverse primer 373R, which is targeted at the highly conserved tRNAile gene. Only some Prochlorococcus strains contained one mismatch. Most deposited cyanobacterial sequences also had a perfect match with the ITS3R primer sequence. Only a number of picocyanobacteria did not contain the target sequence. A BLAST search performed on the reverse primer sequences resulted in highest similarity scores for cyanobacteria. However, it cannot be concluded that the reverse primers are selective for cyanobacteria, since the database of rRNA ITS and 23S sequences is relatively small (compared to 16S sequences) and may be biased for cyanobacteria.
Theoretical melting curves calculated for rRNA-ITS sequences of several major genera (using the program MELT94), showed that the GC clamp positioned at the forward (5') primer yielded a favorable melting curve with a lower melting domain at the 3'-end.
In vitro specificity and applicability of the primers for rRNA-ITS amplification. The forward primer presented in Table 2 had a complete or almost complete match with cyanobacterial sequences, but also some sequence similarity with several non-cyanobacterial 16S rRNA sequences in the database. To confirm the selectivity of the primers for amplification of cyanobacterial rRNA-ITS, we tried to amplify DNA from bacterial strains with some degree of sequence homology to the forward primer (4 to 5 mismatches), and from a few randomly chosen strains. No PCR products were generated from these bacteria, neither at the optimized annealing temperature (as described below), nor 10 degrees below this temperature.
The phylogenetic coverage within the cyanobacterial phylum of the primers was investigated by using DNA from strains of a range of different cyanobacterial genera as template for PCR amplification (Table 3). For each primer combination, the highest temperature at which PCR products were generated for all genera was selected as optimal PCR amplification temperature. As a consequence, for several genera amplification is possible at higher annealing temperatures then those used in our optimized protocol. The number of PCR products and their sizes and relative intensities varied between the different genera (Table 3). For all strains that were tested, ITSa amplification yielded one band of 275 to 350 bp. ITSb amplification resulted in PCR products between 350 and 800 bp for most tested strains, with the exception of Synechocystis, Leptolyngbya and Lyngbya. One PCR product was formed from the genera Microcystis, Pseudanabaena, Planktothrix, Trichodesmium, Prochlorothrix and Synechococcus, and two PCR products from Aphanizomenon, Anabaena, Anabaenopsis, Gloeotrichia, Nodularia, Nostoc and Cylindrospermopsis. ITSc primers successfully amplified DNA from all strains that were tested, yielding for most genera PCR products ranging between 450 and 900 bp. Prochlorothrix hollandica yielded one very long product of 1050 basepairs., Amplification with ITSb or ITSc primers yielded the same number of bands for most strains, only ITSc amplification of Planktothrix strains produced two bands instead of one.
High-resolution separation of Microcystis strains by means of rRNA-ITS DGGE. The ability to differentiate related organisms by means of DGGE was examined using a number of Microcystis strains that had been isolated from Dutch lakes or obtained from culture collections (see Table 1 for details). The DGGE gels in Figure 1 demonstrate the possibility to separate PCR products obtained with the primerset targeted at the 16S (A), ITSa (B), ITSb (C), and ITSc (D), using DNA from 20 selected Microcystis strains. The 16S rRNA PCR product of approximately 450 basepairs gave rise to two bands in the DGGE gel, occupying identical positions for all strains (Fig. 1A). The occurrence of two bands can be explained by the presence of degenerate bases in the non GC-clamped reverse primer (Table 2). In contrast, ITSa amplicons (approximately 325 bp) resulted for most of our isolated strains and all culture collection strains in one DGGE band, for many strains on a unique position (Fig. 1B). Examples of strains with more than one band are V40 with three bands (lane 20, the upper band is not on the figure), and V88 (lane 19) with four bands. These profiles containing two or more bands were encountered predominantly in Microcystis cultures that had been established by isolating single colonies. Compared to plating, this isolation strategy is more likely to yield cultures that are not yet unialgal. The upper bands in lanes 19 and 20 resulted from heteroduplex formation (15) as was confirmed through excision, reamplification and DGGE analysis of these bands. The lower two bands in these cultures were both derived from Microcystis strains as was revealed by a BLAST search of the sequences obtained from these bands.
Results of the DGGE analysis of ITSb and ITSc amplicons from the selected Microcystis strains were largely comparable. Amplification yielded single bands of approximately 500 and 550 basepairs for ITSb and ITSc respectively. This resulted in one band on DGGE gels (Fig. 1C and D), except for strains SAG17.85 (lane 1), K29 (lane 7) and K50 (lanes 8). The positions in the gel were unique for most strains, and, importantly, a number of strains that could not or hardly be distinguished based on ITSa DGGE were separated using ITSb or ITSc DGGE. For example, ITSa amplicons from strains CYA228 (lane 10), PCC7820 (lane 11) and PCC7806 (lane 12) were undistinguishable and differed only faintly from those of strain V91 (lane 13) (Fig. 1B), whereas ITSb or ITSc amplicons enabled a clear separation and left only CYA228 and PCC7820 undifferentiated (Fig. 1C and 1D). Three cultures, SAG17.85 (lane 1), K29 (lane 7), and K50 (lane 8), had multiple bands when analyzed using ITSb or ITSc DGGE, whereas ITSa DGGE had revealed only one band (Fig. 1B-D). Again, the upper bands were shown to result from heteroduplex formation, and all excised lower bands were confirmed to represent Microcystis strains. In contrast to the improved strain differentiation obtained by ITSb or ITSc DGGE compared to ITSa DGGE, examples were found for the opposite situation. For instance, strains PCC7806 (lane 12) and V28 (lane 17), could hardly be distinguished when analyzing ITSb or ITSc amplicons, but occupied clearly different positions in the DGGE gel when ITSa amplicons were used (Fig. 1B-D). The two strains that had yielded two bands on ITSa DGGE, V88 (lane 19) and V40 (lane 20), gave rise to a single band using ITSb or ITSc DGGE (Fig. 1B-D).
The presence of several homologous DNA templates may affect PCR amplification and result in heteroduplex (see above) and chimera formation. To investigate the occurrence of these phenomena we mixed ITSa amplification products from 7 Microcystis strains that could be separated using DGGE, and used these mixtures as templates for ITSa amplification. Figure 2 shows the DGGE banding patterns generated from template mixtures of 2, 3, 5, or 7 Microcystis strains. In spite of the presence of several related sequences, each strain in the mixtures gave rise to one single band of similar intensity, and no additional bands were detected.
DGGE profiles of rRNA-ITS from different cyanobacterial genera. The developed protocols that proved to be effective for Microcystis separation were applied for DGGE analysis of specifically amplified rRNA-ITS of other cyanobacterial genera (Table 3). For almost every strain that was tested, ITSa amplification gave rise to one sharp band, mostly occupying a unique position in the gel (Fig. 3A). Only Aphanizomenon gracile yielded two bands. Also, the longer amplification products of ITSb and ITSc were analyzed using DGGE (Fig. 3B). The patterns for ITSb were mostly similar to those of ITSc and are therefore not shown. Strains from the genera Synechococcus, Synechocystis, Lyngbya, Pseudanabaena, Trichodesmium, and Prochlorothrix, yielded one band (Fig. 3). Leptolyngbya had a second band of lower intensity. Strains from the genera Anabaena, Aphanizomenon, Planktothrix, Cylindrospermopsis, and Gloeotrichia yielded two to four clear bands in most cases, and a few less distinct additional bands for Anabaenopsis. Possibly, some of the bands resulted from heteroduplex formation. Nostoc and Nodularia strains yielded two very fuzzy bands. In addition to the strains shown in Figure 3, a Microcoleus sp. isolate was analyzed and yielded sharp, single bands both on ITSa and ITSc DGGE gels (data not shown).
Field samples. To confirm that the developed methods for high-resolution analysis of cyanobacteria are valid for analysis of the complex microbial communities encountered in the field, we tested two rRNA-ITS primersets on field samples. Samples from three freshwater lakes were taken in summer when dense blooms of cyanobacteria of various genera were observed, and from one lake also in winter when there was no visible presence (including microscopic) of distinctive cyanobacteria. When analyzed on DGGE, ITSa and ITSc PCR products gave rise to complex banding patterns. As shown in the ITSa profiles from Fig. 4., unique as well as (probably) similar genotypes could be identified. The dominant bands were excised from the gel and the PCR products were re-amplified and sequenced. A BLAST search performed on these sequences revealed that the retrieved bands were all derived from cyanobacteria of various genera, including Microcystis, Anabaena, Synechococcus, Oscillatoria and Nostoc.

### DISCUSSION

There are two main considerations for the use of primers specific for cyanobacteria in DGGE analyses. Firstly, specific primers prevent amplification of the abundant DNA of non-cyanobacterial microbes in field samples. The resulting DGGE profiles are less complex compared to those generated with general bacterial primers, hence detection of cyanobacteria that are less abundant or have lower amplification efficiency is feasible. Secondly, characterization of cultures by DGGE, but also by RFLP or sequencing, is not possible when DNA from contaminants is co-amplified. Rendering cyanobacteria axenic can be a difficult and time-consuming procedure, and often they are cultivated more easily when accompanied by heterotrophic bacteria (27). The selectivity of our developed primersets was confirmed through homology search in databases, and through in vitro tests using DNA from cultures of heterotrophic bacteria and from complex microbial communities in freshwater lakes (Fig. 4). The appearance of only 1 to 4 distinct bands in DGGE profiles of cultures from various genera (Fig. 1 and 3) may serve as additional support for selectivity of the developed primersets. None of the cultures used in our study was axenic, yet no unexplainable additional bands were detected on DGGE, even when PCR conditions were made less selective (by substantially lowering annealing temperatures).
Primers 322, described by Wilmotte et al. (40), and 16CITS, described by Neilan et al (26), are targeted at sequences just upstream from the target sequence of forward primer CSIF, and thus could produce virtually identical amplification products. Primer 322 however, is targeted at highly conserved bacterial sequences and is therefore only suitable for studies of axenic cultures of cyanobacteria (14, 21). Primer 16CITS is specific for cyanobacteria. However, from our alignment of 16S sequences it appeared that primer CSIF has more sequence differences with non-cyanobacterial sequences, especially at the 3'site. For primer 16CITS we found 71 non-cyanobacterial sequences with 2 mismatches (as opposed to 0 for primer CSIF), and 266 sequences with 3 mismatches (as opposed to 2 for CSIF). Primer CSIF can therefore be considered more specific for cyanobacteria. Nevertheless, we emphasize that this was based on sequences in the current database and no experimental comparisons between the primers were made.
Coverage of a broad range of genera of primer CSIF was confirmed by software tool Probe Match using published cyanobacterial sequences, and was supported by in vitro tests (Table 3 and Fig. 3). We were able to generate PCR products from Nostoc strain CYA124, even as some deposited sequences from this genus had 3 mismatches with the primer sequence. In any case, PCR products can be formed from all strains from the genera Nostoc and Spirulina (2 or 3 mismatches in deposited sequences), from Gloeobacter and Calothrix (3 mismatches), by including specific primers for these genera, i.e. reducing the mismatches to two or less. The generation of amplification products from strains of all tested cyanobacterial genera when using primer 373R as reverse primer, and from most genera when using primer ITS3R, is supported by the universal occurrence of the highly conserved gene for tRNAile and of certain structural elements in cyanobacteria (7, 13, 34).
The difference in resolution using 16S and ITS DGGE in Microcystis is in agreement with the reported average sequence diversity of less than 1% in 16S (7, 29) compared to up to 7% in rRNA-ITS (26, 30). The high resolution and the single bands that are generated make rRNA-ITS DGGE a valuable method for analysis of this genus. We made the assumption that the multiple bands containing Microcystis sequences in the rRNA-ITS DGGE profiles of some cultures reflect the co-isolation of two different Microcystis strains, instead of the occurrence of two different rRNA operons. This assumption can be confirmed unequivocally by repeated plating to make certain that the analysed cultures are unialgal. This procedure was not carried out here. In any case, the possibility of co-isolation of two morphologically indistinguishable organisms is more likely than the occurrence of multiple different operons in a limited number of deviant strains. Also, all strains that were most confidently unialgal (because they originated from culture collections or had been isolated using plating techniques) gave rise to one band on DGGE. Following this line of reasoning, the two sequences in culture collection strain SAG 17.85 (Fig. 1C and D) have to be explained by unnoticed contamination during years of cultivation in the lab. Support for the occurrence of only one operon (or several identical operons) in the genus Microcystis comes from the unambiguous sequences that Otsuka et al (30) obtained when sequencing the rRNA-ITS of 47 Microcystis strains.
The occurrence of multiple operons in one organism, resulting in rRNA-ITS amplicons of different sizes (Table 3) has been found before in the genera Anabaena, Aphanizomenon, Anabaenopsis, Nostoc, Cylindrospermopsis, and Nodularia (13, 14, 26). Sequencing of the rRNA-ITS from Nodularia, Nostoc, Calothrix and Scytonema strains (3, 7, 13), revealed that not all rRNA operons of cyanobacteria contain tRNAile genes. However, since other operons in these organisms do contain tRNAile genes, they are detectable using ITSa amplification (as shown for Nodularia and Nostoc, Fig. 3A). The presence of tRNA genes on only one copy of the rRNA may explain for a number of genera the production of only one band after ITSa amplification (Fig. 3A and Table 3) despite the existence of different operons as revealed by ITSb or ITSc amplification (Fig. 3B and Table 3). Alternatively, generation of a single ITSa DGGE band could be explained by identical sequences at the 5'-end of different rRNA-ITS operons.
For the generation of DGGE profiles from longer stretches of rRNA-ITS, primerset ITSc is preferable in most cases. Compared to ITSb analysis the resolution was similar (compare Figs 1C and 1D), yet more genera could be amplified and there is more sequence information contained in the amplicons (60-100 bp extra, see Table 3). Nevertheless, for some genera primerset ITSb was more suitable for DGGE profiling. For instance, ITSb amplification products of Nodularia yielded sharp DGGE bands (data not shown), whereas ITSc DGGE resulted in diffuse bands, unsuitable for analysis (Fig. 3B). Also, DGGE profiles resulting from ITSb amplication of Planktothrix aghardii were relatively simple (1 band) compared to the profile resulting from ITSc amplification (3 bands). Due to the occurrence of multiple rRNA-ITS operons in one organism, identification of all dominant bands produced with ITSb and ITSc primers is necessary to come to a reliable estimate of the diversity of cyanobacteria in complex DGGE profiles.
It has to be emphasized that DGGE diversity profiles do not necessarily reflect the true diversity in the field. DNA extraction efficiency and the number of rRNA operons may vary between genera, and the PCR step has several inherent pitfalls, which complicate the interpretation of DGGE profiles of communities. Artefacts introduced by the PCR amplification reaction of homologous sequences include chimera (17) and heteroduplex formation (15) which may lead to an overestimation of the number of organisms, and template annealing (36) and preferential amplification of some DNA templates (33) which may result in a shift in the apparent ratio between organisms after PCR amplification. Heteroduplex bands were formed in some cultures suspected of containing more than one Microcystis isolate (Fig. 1), and in some organisms containing multiple operons (Fig. 3). In contrast, deliberate mixing of template DNA from several Microcystis strains showed no evidence of chimera or heteroduplex formation, since no additional bands were detected in the DGGE profiles (Fig. 2). Equal band intensities pointed to the absence of preferential amplification. However, this effect is more likely between genera since primer binding may differ due to imperfect matching.

### References of example 1

1. Abed, R. M. M., and F. Garcia-Pichel. 2001. Long-term compositional changes after transplant in a microbial mat cyanobacterial community revealed using a polyphasic approach. Environ. Microbiol. 3:53-62.
2. Altschul, S. F., T. L. Madden, A. A. Schaffer, J. H. Zhang, Z. Zhang, W. Miller, and D. J. Lipman. 1997. Gapped BLAST and PSI-BLAST: a new generation of protein database search programs. Nucleic Acids Res. 25:3389-3402.
3. Barker, G. L. A., P. K. Hayes, S. L. O'Mahony, P. Vacharapiyasophon, and A. E. Walsby. 1999. A molecular and phenotypic analysis of Nodularia (cyanobacteria) from the Baltic Sea. J. Phycol. 35:931-937.
4. Becker, S., M. Fahrbach, P. Boger, and A. Ernst. 2002. Quantitative tracing, by Taq nuclease assays, of a Synechococcus ecotype in a highly diversified natural population. Appl. Environ. Microbiol. 68:4486-4494.
5. Beltran, E. C., and B. A. Neilan. 2000. Geographical segregation of the neurotoxin-producing cyanobacterium Anabaena circinalis. Appl. Environ. Microbiol. 66:4468-4474.
6. Bittencourt-Oliveira, M. D., M. C. de Oliveira, and C. J. S. Bolch. 2001. Genetic variability of Brazilian strains of the Microcystis aeruginosa complex (Cyanobacteria/Cyanophyceae) using the phycocyanin intergenic spacer and flanking regions (cpcBA). J. Phycol. 37:810-818.
7. Boyer, S. L., V. R. Flechtner, and J. R. Johansen. 2001. Is the 16S-23S rRNA internal transcribed spacer region a good tool for use in molecular systematics and population genetics? A case study in cyanobacteria. Mol. Biol. Evol. 18:1057-1069.
8. Burger Wiersma, T., L. J. Stal, and L. R. Mur. 1989. Prochlorothrix hollandica Gen-Nov, Sp-Nov, a filamentous oxygenic photoautotrophic procaryote containing chlorophyll a and chlorophyll b - assignment to Prochlorotrichaceae Fam-Nov and Order Prochlorales Florenzano, Balloni, and Materassi 1986, with emendation of the ordinal description. Int. J. Syst. Bacteriol. 39:250-257.
9. Chorus, I., and J. Bartram. 1999. Toxic cyanobacteria in water. E & FN Spon, London.
10. De Rijk, P., and R. De Wachter. 1993. DCSE v2.54, an interactive tool for sequence alignment and secondary structure research. Comput. Applic. Biosci 9:735-740.
11. Garcia-Pichel, F., M. Kühl, U. Nübel, and G. Muyzer. 1999. Salinity-dependent limitation of photosynthesis and oxygen exchange in microbial mats. J. Phycol. 35:227-238.
12. Hall, T. A. 1999. BioEdit: a user-friendly biological sequence alignment editor and analysis program for Windows 95/98/NT. Nucl. Acids. Symp. Ser. 41:95-98.
13. Iteman, I., R. Rippka, N. Tandeau de Marsac, and M. Herdman. 2000. Comparison of conserved structural and regulatory domains within divergent 16S rRNA-23S rRNA spacer sequences of cyanobacteria. Microbiology 146:1275-1286.
14. Iteman, I., R. Rippka, N. Tandeau de Marsac, and M. Herdman. 2002. rDNA analyses of planktonic heterocystous cyanobacteria, including members of the genera Anabaenopsis and Cyanospira. Microbiology 148:481-496.
15. Jensen, M. A., and N. Straus. 1993. Effect of PCR conditions on the formation of heteroduplex and single-stranded DNA products in the amplification of bacterial ribosomal DNA spacer regions. PCR-Methods Appl. 3:186-194.
16. Jonkers, H. M., R. Ludwig, R. De Wit, O. Pringault, G. Muyzer, H. Niemann, N. Finke, and D. De Beer. 2003. Structural and functional analysis of a microbial mat ecosystem from a unique permanent hypersaline inland lake: 'La Salada de Chiprana' (NE Spain). FEMS Microbiol. Ecol. 1488:1-15.
17. Kopczynski, E. D., M. M. Bateson, and D. M. Ward. 1994. Recognition of chimeric small-subunit ribosomal DNAs composed of genes from uncultivated microorganisms. Appl. Environ. Microbiol. 60:746-748.
18. Kurmayer, R., E. Dittmann, J. Fastner, and 1. Chorus. 2002. Diversity of microcystin genes within a population of the toxic cyanobacterium Microcystis spp. in Lake Wannsee (Berlin, Germany). Microb. Ecol. 43:107-118.
19. Kwok, S., D. E. Kellog, N. McKinney, D. Spasic, L. Goda, C. Levenson, and J. J. Sninsky. 1990. Effects of primer-template mismatches on the polymerase chain reaction: Human immunodeficiency virus 1 model studies. Nucleic Acids Res 18:999-1005.
20. Laamanen, M. J., L. Forsstrom, and K. Sivonen. 2002. Diversity of Aphanizomenon flos-aquae (cyanobacterium) populations along a Baltic Sea salinity gradient. Appl. Environ. Microbiol. 68:5296-5303.
21. Laloui, W., K. A. Palinska, R. Rippka, F. Partensky, N. Tandeau de Marsac, M. Herdman, and I. Iteman. 2002. Genotyping of axenic and non-axenic isolates of the genus Prochlorococcus and the OMF-'Synechococcus' clade by size, sequence analysis or RFLP of the Internal Transcribed Spacer of the ribosomal operon. Microbiology 148:453-465.
22. Lovell, C. R., M. J. Friez, J. W. Longshore, and C. E. Bagwell. 2001. Recovery and phylogenetic analysis of nifH sequences from diazotrophic bacteria associated with dead aboveground biomass of Spartina alterniflora. Appl. Environ. Microbiol. 67:5308-5314.
23. Lu, W. Q., E. H. Evans, S. M. McColl, and V. A. Saunders. 1997. Identification of cyanobacteria by polymorphisms of PCR- amplified ribosomal DNA spacer region. FEMS Microbiol. Lett. 153:141-149.
24. Maidak, B. L., J. R. Cole, T. G. Lilburn, C. T. Parker, P. R. Saxman, R. J. Farris, G. M. Garrity, G. J. Olsen, T. M. Schmidt, and J. M. Tiedje. 2001. The RDP-II (Ribosomal Database Project). Nucleic Acids Res. 29:173-174.
25. Muyzer, G., E. C. de Waal, and A. G. Uitterlinden. 1993. Profiling of complex microbial populations by denaturing gradient gel electrophoresis analysis of polymerase chain reaction-amplified genes coding for 16S rRNA. Appl. Environ. Microbiol. 59:695-700.
26. Neilan, B. A., J. L. Stuart, A. E. Goodman, P. T. Cox, and P. R. Hawkins. 1997. Specific amplification and restriction polymorphisms of the cyanobacterial rRNA operon spacer region. Syst. Appl. Microbiol. 20:612-621.
27. Nübel, U., F. GarciaPichel, and G. Muyzer. 1997. PCR primers to amplify 16S rRNA genes from cyanobacteria. Appl. Environ. Microbiol. 63:3327-3332.
28. Orcutt, K. M., U. Rasmussen, E. A. Webb, J. B. Waterbury, K. Gundersen, and B. Bergman. 2002. Characterization of Trichodesmium spp. by genetic techniques. Appl. Environ. Microbiol. 68:2236-2245.
29. Otsuka, S., S. Suda, R. H. Li, M. Watanabe, H. Oyaizu, S. Matsumoto, and M. M. Watanabe. 1998. 16S rDNA sequences and phylogenetic analyses of Microcystis strains with and without phycoerythrin. FEMS Microbiol. Lett. 164:119-124.
30. Otsuka, S., S. Suda, R. H. Li, M. Watanabe, H. Oyaizu, S. Matsumoto, and M. M. Watanabe. 1999. Phylogenetic relationships between toxic and non-toxic strains of the genus Microcystis based on 16S to 23S internal transcribed spacer sequence. FEMS Microbiol. Lett. 172:15-21.
31. Palinska, K. A., W. Liesack, E. Rhiel, and W. E. Krumbein. 1996. Phenotype variability of identical genotypes: The need for a combined approach in cyanobacterial taxonomy demonstrated on Merismopedia-like isolates. Arch. Microbiol. 166:224-233.
32. Rasmussen, U., and M. M. Svenning. 2001. Characterization by genotypic methods of symbiotic Nostoc strains isolated from five species of Gunnera. Arch. Microbiol. 176:204-210.
33. Reysenbach, A. L., L. J. Giver, G. S. Wickham, and N. R. Pace. 1992. Differential amplification of ribosomal-RNA genes by Polymerase Chain Reaction. Appl. Environ. Microbiol. 58:3417-3418.
34. Rocap, G., D. L. Distel, J. B. Waterbury, and S. W. Chisholm. 2002. Resolution of Prochlorococcus and Synechococcus ecotypes by using 16S-23S ribosomal DNA internal transcribed spacer sequences. Appl. Environ. Microbiol. 68:1180-1191.
35. Rychlik, W. 1995. Selection of primers for polymerase chain-reaction. Mol. Biotechnol. 3:129-134.
36. Suzuki, M. T., and S. J. Giovannoni. 1996. Bias caused by template annealing in the amplification of mixtures of 16S rRNA genes by PCR. Appl. Environ. Microbiol. 62:625-630.
37. Tillett, D., and B. A. Neilan. 2000. Xanthogenate nucleic acid isolation from cultured and environmental cyanobacteria. J. Phycol. 36:251-258.
38. Whitton, B. A., and M. Potts. 2000. The ecology of cyanobacteria, their diversity in time and space, 1st ed. Kluwer Academic Publishers, Dordrecht, the Netherlands.
39. Wilmotte, A. 1994. Molecular evolution and taxonomy of the cyanobacteria, p. 1-25. In D. A. Bryant (ed.), The molecular biology of cyanobacteria. Kluwer Academic Publishers, Dordrecht, The Netherlands.
40. Wilmotte, A., G. Van der Rauwera, and R. De Wachter. 1993. Structure of the 16-S Ribosomal-RNA of the thermophilic cyanobacterium Chlorogloeopsis HTF (Mastigocladus-Laminosus HTF) Strain PCC 7518, and phylogenetic analysis. FEBS Lett. 317:96-100.
41. Young, J. P. W. 1992. Phylogenetic classification of nitrogen-fixing organisms, p. 43-86. In G. Stacey, H. J. Evans, and R. H. Burris (ed.), Biological Nitrogen Fixation. Chapman & Hall, New York.
42. Zehr, J. P., M. T. Mellon, and W. D. Hiorns. 1997. Phylogeny of cyanobacterial nifH genes: Evolutionary implications and potential applications to natural assemblages. Microbiology-(UK) 143:1443-1450.
43. Zwart, G., W. D. Hiorns, B. A. Methe, M. P. Van Agterveld, R. Huismans, S. C. Nold, J. P. Zehr, and H. J. Laanbroek. 1998. Nearly identical 16S rRNA sequences recovered from lakes in North America and Europe indicate the existence of clades of globally distributed freshwater bacteria. Syst. Appl. Microbiol. 21:546-556.

### Figure legends of example 1

FIG. 1. Discrimination of closely related cyanobacteria. DNA from 20 Microcystis strains was amplified with 4 different primer combinations (A-D) and separated on DGGE gels. Primer sequences and combinations are given in Table 2. The different primer combinations amplified a segment of the 16S gene (A) or different portions of the rRNA-ITS, ITSa (B), ITSb (C), or ITSc (D). The Microcystis strains are SAG17.85 (1), V80 (2), V72 (3), V73 (4), CYA43 (5), V67 (6), K29 (7), K50 (8), CYA140 (9), CYA228 (10), PCC7820 (11), PCC7806 (12), V91 (13), Z6 (14), Z11 (15), V89 (16), V28 (17), S2 (18), V88 (19), V40 (20). Information about the Microcystis strains is given in Table 1. The DGGE gels had a 30-40% denaturant concentration gradient and only the part of the gel containing bands is shown.
FIG. 2. DGGE profiles of ITSa amplified from DNA template mixtures. Lane 1-4 show ITSa PCR products which were amplified from equal concentrations of template mixtures of Microcystis strains PCC7820 + K29 (1), PCC7820 + K29 + SAG17.85 (2), Z6 + PCC7820 + CYA140 + K29 + SAG17.85 (3), and S2 +Z6 + PCC7820 + CYA140 + K29 + CYA43 + SAG17.85 (4).
FIG. 3. DGGE profiles of various cyanobacterial genera amplified with ITSa and ITSc primers. The following strains were analyzed: Synechococcus sp. (1), Synechocystis sp. (2), Leptolyngbya sp. (3), Lyngbya sp. (4), Pseudanabaena catenata (5), Trichodesmium erythraeum (6), Anabaena variablis (7), Aphanizomenon flos-aquae (8), Aphanizomenon gracile (9), Planktothrix aghardii (10), Planktothrix prolifica (11), Cylindrospermopsis raciborskii (12), Anabaenopsis arnoldi (13), Gloeotrichia sp. (14), Nostoc sp. (15), Nodularia spumigena (16), Prochlorothrix hollandica (17). The gels had a denaturant concentration gradient of 25-40% and only the part of the gel containing bands is shown. The lower gel was assembled from two gels.
FIG. 4. DGGE profiles generated from ITSa amplification products from freshwater lake samples. The samples were taken from Lake Zeegerplas on July 20 (lane 1) and August 21 (lane 2), both when cyanobacteria were blooming, and from Lake Kinselmeer on May 5 (lane 3) during a cyanobacterial bloom (lane 3), and on January 16 in the absence of a visible presence of cyanobacteria (lane 4). Several dominant bands were excised, re-amplified and sequenced.

### Example 2

We show that genetic differentiation of Microcystis colonies based on rRNA-ITS sequences correlates with microcystin production. Hence, ecological studies of toxic and non-toxic cyanobacteria are now feasible through studies of rRNA-ITS genotypic diversity in isolated cultures or colonies, and in natural communities. Microcystis colonies (107) were isolated from 14 European and 1 Maroccan lakes, the presence of microcystins in each colony was examined by MALDI-TOF, and they were grouped using rRNA-ITS DGGE. Based on DGGE analysis of amplified ITSa and ITSc fragments, yielding supplementary resolution (Janse et al., 2003), 59 classes could be distinguished. Microcystin-producing and non-producing colonies were separated into different classes. Sequences obtained from at least one representative strain from most classes were congruent with the classification based on rRNA-ITS DGGE. Alignment of these colony sequences and published rRNA-ITS sequences from cultured Microcystis strains of known toxicity confirmed that microcystin production correlated with rRNA-ITS sequences. About 30% of the analyzed colonies gave rise to more than one band in DGGE profiles, indicating either aggregation of different colonies or the occurrence of sequence differences between multiple operons in some strains. Several colonies sharing identical rRNA-ITS sequences were assigned to different morphospecies, and colonies of one morphospecies contained various rRNA-ITS sequences, confirming the inconsistencies reported for Microcystis identification based on morphology. There was no indication for geographical restriction of strains since identical sequences could originate from geographically distant lakes. DGGE community profiles from two Dutch lakes from which colonies had been isolated showed abundance of different strains at subsequent bloom stages. Although not all bands in the community profiles could be matched with isolated colonies, the profiles suggest that non-toxic colonies dominate later in the season and in scums.

### INTRODUCTION

Worldwide, eutrophic freshwater lakes endure noxious blooms and surface scums caused by cyanobacteria from the genus Microcystis. During many blooms, Microcystis cells produce the hepatotoxin microcystin which is toxic to humans and many other eukaryotes (3).
Microcystins are cyclic heptapeptides, formed non-ribosomally by peptide- and polyketide-synthetases. More than 70 structural microcystin variants are known to date (12).

We provide rapid and reliable methods for toxin measurements that allow for a proper assessment of the hazards that Microcystis blooms pose on human health and aquatic ecosystems. Predicting how toxin concentrations will develop, requires tools and insights for understanding the dynamics of microcystin production. Environmental factors may affect microcystin production in Microcystis cultures by a factor 3-4 (23). However, the actual capability of microcystin production is genetically determined. Microcystin producing and non-producing strains coexist in natural populations (6, 28). Strains isolated from the same bloom sample are constitutively toxic or non-toxic (14, 19, 28) and the cellular microcystin content may differ considerably between strains (2). The decisive factor determining the average cellular microcystin content in a given natural population, and thereby the toxicity of a bloom, is the abundance of microcystin producing strains (4).

Understanding of the community composition and dynamics of microcystin producing and non-producing Microcystis strains in the field is very limited, due to a lack of suitable identification methods. Traditional characterization of Microcystis based on morphological features is very difficult, moreover, transition between morphological characteristics has been observed (20). Identification difficulties may partly explain why correlations between morphology and toxicity have proven to be unreliable (6, 13, 21, 29), emphasizing the need for identification independent of morphological characteristics. Molecular biological methods provide tools for recognition of Microcystis strains that are more reliable, and the high resolution that can be attained makes differentiation below genus level possible.

A number of studies have targeted the microcystin synthetase (mcy) gene cluster (5, 25), for identification of toxic Microcystis strains. This approach has the obvious advantage of a direct relationship between gene detection and toxicity, provided non-toxic strains do not possess the gene. Detection by PCR of the mcyB gene, the mcyA gene, or the adenylation domain within the microcystin synthatase gene cluster, showed good correlation with toxin production, but some anomalies were found. A small number of Microcystis strains contained the mcy gene cluster but lacked detectable microcystins (10, 15, 17, 18, 26). The high sequence similarity between the mcyB region and other peptide synthetase loci (5, 26) and the occurrence of multiple adenylation domains in toxic and non-toxic Microcystis spp. (18) may explain these discrepancies. Alternatively, Nishizawa et al. (18) suggested that some non-toxic Microcystis strains do contain the mcyABC gene. To avoid the biases and limitations inherent to studies on cultured isolates, genetic analyses have also been done on colonies that had been isolated directly from the field. Kurmayer et al. (12) separated Microcystis colonies from a field population in size classes and showed through quantification of genotypes with and without the mcyB gene that larger colonies were mainly responsible for microcystin production. In a survey of a large number of (non-cultivated) Microcystis colonies that had been characterized for microcystin production using matrix-assisted laser desorption/ionization time-of-flight mass spectrometry (MALDI TOF MS) (6), Microcystis specific primers targeted at mcyB (13) did not yield PCR products from 39% of microcystin containing colonies (false negatives). However, combination with a diagnostic PCR targeted at mcyA, resulted in only a few (4.9%) false negatives, as well as a few (2.5%) false positives (29).

In the present invention, we used an alternative approach to characterize toxic and non-toxic Microcystis colonies in natural populations, independent of mcy gene detection. Isolated colonies in which the presence of microcystins had been examined were differentiated at high resolution based on sequences of a 'taxonomic' marker gene (rRNA-Internal Transcribed Spacer, rRNA-ITS) known to differ considerably between Microcystis strains (9, 21). We show that analysis of the rRNA ITS by using denaturing gradient gel electrophoresis (DGGE) or by sequencing, is sufficiently resolving for identification of toxic and non-toxic Microcystis strains in single isolated colonies and in natural samples.

Because strain differentiation was based on sequence differences in universal genes, the pitfalls of diagnostic PCR when using mcy gene detection (occurrence of false positives and negatives) were avoided. Moreover, this approach provides ecologically relevant insights into the dynamics of all cyanobacterial strains that are present in a natural community. Instead of focusing on the limited subgroup of mcy gene-containing Microcystis strains, different toxic and non-toxic strains can be distinguished. Finally, since colony properties other than microcystin production (such as the production of other - potentially toxic- peptides) may also correlate with rRNA-ITS classification, this gene offers possibilities for characterization of a broader range of strain properties. It is preferred that all strains that can be encountered are characterized with regard to the strain properties of interest (such as toxin production) and rRNA-ITS sequence information.

### Materials and methods

### Sampling and isolation of colonies

Sampling for colony isolation was carried out during summer 2001 in 15 water bodies from 9 European countries and Marocco by net hauls using a plankton net (40µm mesh size). The samples were kept cool until they were processed. Colony isolation and morphological description were standardized in the course of a workshop held from 2-7 July 2001 in Berlin, see also (29). For colony isolation, samples were diluted and single colonies picked out by using thin Pasteur pipettes or forceps under binocular microscopes. Colonies were washed in BG11 medium (22) to dispose of any visible secondary colonies or other cyanobacteria. Morphological classification was done using the morphological criteria proposed by Komarek and Anagnostidis (11). Colonies were transferred into a reaction tube (0.5ml) containing culture medium (final sample volume 4-30 µl) and the presence of each colony in the tube was verified microscopically. The tubes were frozen (liquid nitrogen) and thawed several times to disintegrate the colonies, and stored at -20°C. Aliquots from each tube were used for gene amplification and for matrix-assisted laser desorption/ionization time-of-flight mass spectrometry (MALDI-TOF MS).

### MALDI-TOF MS

MALDI-TOF MS measurements were performed as described in (6, 29). 1-4 µl of the cell suspension from each colony was prepared onto a stainless steel template and immediately 1 µl of matrix (10 mg/ml 2,5-dihydroxybenzoic acid in water/acetonitrile (1:1) with 0.03% trifluoroacetic acid) was added. Positive ion mass spectra were recorded from each colony using a MALDI-TOF mass spectrometer (Voyager DE-PRO, PerSeptive Biosystems, Framingham, MA) equipped with a reflectron. For desorption of the components a nitrogen laser beam (1 = 337 nm) was focused on the template. The acceleration voltage was set at 20 kV. Measurements were performed in the delayed extraction mode, allowing the determination of monoisotopic mass values. A low mass gate of 500 improved the measurement by filtering out the most intensive matrix ions. Chlorophyll-a degradation products phaeophytine-a and phaeophorbide-a with mass values of m/z 871.57 and 593.27 Da were used for internal calibration. After determination of monoisotopic mass values microcystins were identified by Post Source Decay (PSD) measurements of fragment ions. The precursor ions were selected with a Time Ion Selector having a mass window of 10 mass units. The operating voltages of the reflectron were reduced stepwise to record 12 spectral segments sequentially. PSD analysis were performed directly from the same colony on the template.

### Sampling and DNA Isolation for field profiles

Lakes 't Joppe and Zeegerplas are eutrophic lakes (maximum depth approximately 25 m) which are stratified during the summer. Water samples were collected 0.5 m below the surface, or from surface scum, in sterile bottles from a boat in the middle of the lake and stored in the dark at 4°C. Within four hours after sampling, a volume of 250 ml water or less if the filter clogged, was filtered over a 25 mm diameter, 0.2 mm pore size mixed esters filter (ME 24, Schleicher & Schuell, Dassel, Germany). The filter was cut in two with a sterile scalpel and each half was stored in a microcentrifuge tube at -80°C until further processing. DNA from filters containing field samples was isolated as described by Zwart et al. (31).

### PCR amplification

Primer sequences and amplification conditions were according to (9).
Primer CYA 371F (forward primer for nested PCR) had the following sequence: 5'- CCT ACG GGA GGC AGC AGT GGG GAA TTT TCC AC-3'. Primer GC-CSIF (forward primer for ITSa and ITSc amplification) had the following sequence: 5'- GYC ACG CCC GAA GTC RTT AC-3'. Primer 373R (reverse primer for ITSa amplification) had the following sequence: 5'- CTA ACC ACC TGA GCT AAT-3', and primer ULR (reverse primer for nested PCR and for ITSc amplification) had the following sequence: 5'- CCT CTG TGT GCC TAG GTA TC-3'. PCR amplification was performed in an MBS 0.5 S thermocycler (ThermoHybaid, Ashford, UK) in a 25 ml reaction mixture containing approximately 50 ng of DNA, (10 mM Tris/HCl pH 8.3, 50 mM KCl, 0.01% w/v gelatin, 200 mM of each deoxynucleotide, 1.5 mM MgCl2, 2.5 units of Taq DNA polymerase and 0.5 mM of each primer). The temperature cycling conditions for the amplification of rRNA-ITS, ITSa and ITSc (9) were as follows. After a pre-incubation at 94°C for 5 min, a total of 30 cycles were performed of 94°C for 1 min, the annealing temperature Ta for 1 min and 72°C for 1 min. In the first twenty cycles, Ta decreased by 1°C after every second cycle from 62°C in the first cycle to 52°C in the twentieth. This 'touch down' procedure was followed to reduce non-specific annealing of the primers. In the last ten cycles, Ta was 52°C. The temperature cycling was concluded with a final step of 30 min at 72°C.

### DGGE profiling

DGGE was performed essentially as described by Muyzer et al. (16). Briefly, PCR products were separated on a 1.5 mm thick, vertical gel containing 8% (w/v) polyacrylamide (37.5:1 acrylamide: bisacrylamide) and a linear gradient of the denaturants urea and formamide, increasing from 25 or 30% at the top of the gel to 40% at the bottom. The concentrations depended on the gene products that were analyzed and are given in the figure legends. Here, 100% denaturant is defined as 7 M urea plus 40% v/v formamide. Electrophoresis was performed in a buffer containing 40 mM Tris, 40 mM acetic acid, 1 mM EDTA (pH 7.6) (0.5' Tris-acetate-EDTA buffer) for 16 hours at 75 V. The gel was stained for 1 h in water containing 0.5 µg ml-1 ethidium bromide. An image of the gel was recorded with a CCD camera system (Imago, B&L Systems, The Netherlands).

### Sequencing of DNA from DGGE bands

A small piece of gel from the middle of the target band was excised from the DGGE gel and incubated in 50 ml sterile milli-Q purified water for 24 hours at 4°C. The eluent was reamplified using the original primerset and run on DGGE to confirm its identity. PCR products were purified and sequenced using Applied Biosystems 3730 and 3100 genetic analysers by Baseclear Labservices (Baseclear, Leiden, the Netherlands). Sequences were deposited at EMBL and were assigned accession numbers AJ605140-AJ605221 for sequences from isolated colonies. Similarity with sequences deposited in Genbank/EMBL/DDBJ was checked using the program Blast (1)(via
http://www.ncbi.nlm.nih.gov/BLAST/).

### Sequence alignment and investigation

Sequences from the rRNA-ITS region were obtained from Genbank/EMBL/DDBJ and were aligned using the programs ClustalW, the BioEdit Sequence Alignment Editor (7), and the program package ARB (www.ARB-home.de). Trees were constructed from aligned sequences using ARB and Treecon (27).

### RESULTS

### Isolation and characterization of Microcystis colonies

The Microcystis colonies we used for our investigations originated from 15 lakes from 9 European countries and Marocco. Most of the water samples had been collected early July 2001 and were isolated and characterized by experts during a European workshop. Additional colonies from two Dutch lakes ('t Joppe and Zeegerplas) were isolated and characterized in August and September. A study of the relations between colony morphotypes, occurrence of microcystins, and diagnostic PCR targeted at the mcyA and mcyB genes, has been published by Via-Ordorika et al. (29). These authors identified eight morphotypes, mostly M. aeruginosa and M. ichthyoblabe, and less frequently M. botrys, M. flos-aquae, M. panniformis, M. viridis, M. wesenbergii and M. novacekii. A small number of Microcystis colonies exhibited intermediate morphological characteristics. For details on relative abundances and distribution over the sampled lakes of these morphotypes, we refer to their paper. In 123 out of 151 colony samples we obtained, the presence of toxins had been determined by MALDI TOF MS and in 111 of these colonies toxin production capacity had been confirmed by PCR detection of the mcy gene. Eight colonies that had not yielded a MALDI TOF signal, had been characterized by mcy PCR only.

### Grouping of Microcystis colonies based on rRNA-ITS DGGE

To enable grouping of Microcystis colonies based on rRNA-ITS, we amplified DNA fragments that are suitable for DGGE analysis (9) from the small quantities of cells in colony aliquots that remained after the analyses described above. A nested PCR protocol was used to maximize DNA retrieval. In the first step, cyanobacteria specific 16S primer CYA 371F and universal 235 primer ULR were used for amplification of the a major part of the 16S, the ITS, and short section of the 23S of the rRNA gene. The resulting PCR product was diluted and used as template for amplification of the 3'-end of the 16S plus part of the rRNA ITS (ITSa) by using cyanobacteria specific 16S primer CSIF in combination with ITS primer 373R, or for amplification of a longer fragment spanning the entire rRNA ITS (ITSc) by using primers CSIF and ULR (9).

Out of the total of 151 sample aliquots, 107 yielded PCR products. Successful amplification was only slightly biased for lake of origin (between 70-100% of the colonies originating from a lake yielded PCR products, with the exception of 25% from lake Brno), morphospecies assignation (between 60- 85% of the colonies assigned to a morphospecies yielded PCR products with the exception of the two M.viridis colonies) and presence of toxins (75% of both toxic and non-toxic colonies, and 48% of the colonies with undetectable toxins yielded PCR products). Consequently, the collection of colonies that was subjected to DGGE analysis contained Microcystis colonies derived from all 13 water bodies and covering all morphospecies except M. viridis. Toxin data were available for 96 from the 107 colonies that yielded PCR products.

Colonies were differentiated by analysis of ITSa amplicons on DGGE. Based on the position of ITSa bands in the gel (Fig. 1), colonies were assigned to 27 different groups (Table 1). To enable between gels comparison of bands, each new batch of colonies was analyzed together with a mixture of colonies assigned to different groups and thus representing different gel positions. Differentiation of colonies based on ITSc DGGE was more difficult due to the smaller differences in migration of the longer sequences. Nevertheless, colonies could be assigned to 19 different groups based on ITSc DGGE (Table 1). Several of the colonies gave rise to profiles with two or more bands (e.g. Fig 1, colonies #63 or #147). In the 8 profiles with more than two bands, the upper one or two bands were heteroduplexes as was shown through excision, reamplification and analysis on DGGE of these bands. Only one colony (#119) appeared to contain three different (non-heteroduplex) bands in ITSa DGGE profiles. Out of 107 analyzed colonies, 76 yielded ITSa as well as ITSc DGGE profiles containing one sequence (e.g. #65), and 11 colonies yielded ITSa and ITSc profiles containing two sequences (e.g. #63). For 14 colonies, profiles of one ITSa band and two ITSc bands were generated (e.g. #147), whereas 5 colonies showed one ITSc band and two ITSa bands (e.g. #112)(Table 1).

There was no apparent relation between morphospecies and rRNA-ITS DGGE profiles, since colonies from each morphospecies were assigned to different ITS DGGE classes. Also, with the exception of Lake Frederikborg (Denmark), the lakes yielded colonies from multiple ITS DGGE classes. Hence, multiple colonies in an ITS DGGE class were in most cases isolated from different lakes and assigned to different morphospecies (Table 1).

The position of ITSa bands on DGGE was not a sufficient determinant for differentiation of all toxic and non-toxic colonies. In ITSa group 11 for instance, several non-toxic colonies were grouped together with a majority of toxic ones (Table 1). In combination with differentiation based on ITSc DGGE, however, colonies could be sufficiently set apart to allow classification of toxic and non-toxic colonies into distinct groups. Only one colony (#100, see ITSa group 18 and ITSc group 18, denoted as 18a/18c) in which a toxin was identified could not be differentiated from non-toxic colonies on the basis of ITS DGGE. However, toxin identification in this colony (as well as that in colonies #13, 26a/12c, and #112, 4a/15c and 15a/15c) was questionable, since it was based on a MALDI TOF MS peak that could also correspond to another cyanopeptide than Microcystin LR (Table 1). Hence, identification of toxic and non-toxic Microcystis colonies could be achieved on the basis of ITSa plus ITSc DGGE profiles.

### rRNA-ITS sequences from colonies

To substantiate the DGGE-based classification of colonies, and to allow in-depth analysis of the Microcystis classes that were identified, ITSc was sequenced from colonies and bands representative for most of the DGGE classes of Table 1. When possible (e.g. colony #60 and #105 in classes 19a/12c plus 26a/12c and 18a/17c plus 24a/17c respectively), sequencing of two ITSa bands resolved ambiguous base calls resulting from a mixture of sequences that were not separated on ITSc DGGE. Out of 59 ITS DGGE classes, we obtained from 15 classes multiple sequences, from 29 classes one sequence and from 14 classes no sequences. Attempts to obtain sequences from some bands (e.g. from colony #78, class 18a/1c and 18a/2c) failed due to the occurrence of many ambiguous base calls. Most (10) of the classes that were not sequenced contained only one sequence, and for 5 non-sequenced classes there were no toxicity data available.
The sizes of Microcystis ITS ranged between 354 and 362 bp and variation between strains was restricted to approximately 11% of the sequence positions. Different colonies within a DGGE ITS class yielded identical sequences in most cases (e.g. all colonies in class 16a/9c). Nevertheless, a one basepair substitution in the 5' region of the ITS differentiated sequence 12 from 15-1 in class 8a/5c and a substitution in the 16S differentiated 10-u from the other sequences in class 11a15c.
Microcystis rRNA ITS sequences were aligned and phylogenetic trees were constructed based on the alignment of ITSa or ITSc fragments. The ITSa tree revealed that sequence differences in the ITSa region were sufficient for discrimination of toxic and non-toxic colonies (data not shown). The only exception was toxic colony #100 which had an ITS sequence identical to that of non-toxic colonies #87 and #89, which had been classified in the same ITS DGGE class 18a/18c (see above and Table 1). Inclusion in the alignment of the ITSa sequences of 47 Asian Microcystis strains of known toxin production (21), resulted in two occurrences of identical sequences associated with toxic as well as non-toxic Microcystis strains. Toxic colony #39 (27a/19c) had an ITSa sequence identical to 5 non-toxic M. novacekii strains (TAC66, TAC65, TAC20, CC2, BC18), and non-toxic colony ITSa sequences 148 (18a/7c) and 105-u (18a/17c) were identical to two non-toxic M. ichtyoblabe strains (TAC136 and TAC138) but also one toxic M, ichtyoblabe strain (TAC125).

Sequences covering the entire rRNA-ITS were analyzed to resolve these imperfections in the discrimination of toxic and non-toxic strains. The phylogenetic tree based on ITSc alignment (Fig. 3) shows that sequences from the different colony DGGE classes, as well as from different Asian strains, end up in separate branches. All our 107 Microcystis colonies aside from colony #100, as well as the 47 Microcystis strains mentioned above, contained rRNA-ITS sequences with sufficient variation for discrimination of toxin producers and non-producers. Clustering of toxin producers and of non-producers was observed, however, a few toxin producers ended up in clusters withnon-toxic sequences (e.g. #39) and vice versa (e.g. AB015386). Although clustering of Asian Microcystis cultures and European colonies was observed (e.g. AB015362 etc. and BK146), these sequences were never identical.

### Community profiles from original field sample

To investigate to what extent the isolated colonies represented the cyanobacterial community in their lakes of origin, we analyzed ITSa and ITSc DGGE profiles from the water samples from which most colonies had been harvested. These samples had been taken from the water column of Lakes 't Joppe and Zeegerplas in June, August and September 2001, and from the surface scum of 't Joppe at the latter two sampling dates. Ambient abiotic parameters, microcystin concentrations, and cell and colony numbers are given in Table 2.
Microbial communities from these lakes were collected on filters and ITSa and ITSc DGGE profiles were generated (Fig. 2). The number of different sequences that could be distinguished in ITS DGGE community profiles of water samples, was lower than the number that had been identified based on isolated colonies. In ITSa community profile from 't Joppe we detected 12 bands (4 of which less distinct) in the lower part of the gel where Microcystis sequences were expected, and 6 bands in the upper part of the gel, while isolated Microcystis colonies from this lake had been differentiated into 18 groups based on ITSa DGGE (Table 1). In ITSa community profiles from the Zeegerplas, 8 different Microcystis bands (2 less distinct) were discernable, while colony analysis had resulted in the identification of 9 groups. ITSc DGGE yielded for both lakes profiles with 8 bands (1 less distinct) in the lower part of the gels, while colonies isolated from 't Joppe and Zeegerplas had been differentiated in 15 and 7 groups, respectively (Table 1).
For identification of Microcystis strains, bands at different positions in the ITS profiles from water samples were excised, reamplified, and their position and purity were verified on DGGE. Sequences were retrieved from the bands indicated in Fig. 2 and Table 3. Compared to ITSa profiles, retrieval of pure bands for sequencing proved more difficult from ITSc profiles. Greater differences in melting behavior of the smaller ITSa fragments made distinction and isolation of bands easier (and also explains the higher number of discernable bands). ITSa sequences were also sufficiently discriminating for identification of colony toxicity (see above). Nevertheless, we also put an effort in the retrieval of bands from ITSc profiles, mainly to obtain full sequences from strains that become dominant in scums.

A BLAST search revealed that bands e1, e18, e19, e20, and e27, excised from the upper part of the gels were derived from Anabaena, Aphanizomenon and Synechocystis, whereas all other bands contained Microcystis sequences. Sequences from most of the bands excised from ITSa profiles matched with sequences from isolated colonies. From the nine different Microcystis sequences we retrieved, only one (derived from band e3) aligned with sequences corresponding to toxic colonies, five aligned with non-toxic sequences (derived from bands e4= e5= e9, e6= e11, e12= e15, e8 and e17), one (derived from band e 10) with a sequences of unknown toxicity, and two sequences (derived from bands e2= e7= e13= e16 and e14) did not correspond to any isolated colony (Table 3). The most prominent bands that were amplified contained sequences corresponding to non-toxic colonies (e4= e5= e9, e6= e11 and e12= e15) or sequences of unknown toxicity (e10 and e7= e16). From the six different sequences retrieved from ITSc profiles, one (derived from band e28) corresponded to a toxic strain, two (derived from bands e21= e22 and e25= e29) tonon-toxic strains, and three (e23, e24 and e26) yielded new sequences not corresponding to any isolated colony (Table 3). The sequences of two of the strains that became relatively dominant in scums were detected in ITSa profiles (bands e7 and e10) as well as in ITSc profiles (bands e24 and e26).

### DISCUSSION

Our data show that physiological differences between Microcystis strains (microcystin-producing versus non-producing) can be correlated with genetic differences in the rRNA-ITS. Thus, ecological studies of toxic and non-toxic Microcystis strains are possible using colonies isolated from natural samples, cultured isolates, or field samples. Recently, the production of microcystin variants, differing in toxicity to a large extent (E Chorus and Bartram ref, ?), has been found to have a genetic basis (15). Therefore, even information on the relative toxicity of strains could be provided in the rRNA-ITS sequences since a correlation with rRNA-ITS sequences exists.

Genotype analysis using DGGE enables processing of many samples, assessment of the purity of isolated cultures or colonies, and detection of various genotypes in complex natural communities. Differentiation of colonies was achieved by means of ITSa and ITSc DGGE. Although a somewhat better separation of bands could be achieved by ITSa DGGE (Fig. 2), ITSc DGGE yielded supplementary resolution and contaminant detection in several cases, and provided more sequence information (9)(Table 1). DGGE based on ITSa or ITSc alone yielded insufficient resolution for discrimination of toxic and non-toxic Microcystis strains. In combination, however, these methods provided a suitable basis for identification of toxic strains (Table 1).

The partial rRNA-ITS sequences covered by ITSa amplicons discriminated toxic and non-toxic colonies in our dataset. Therefore, sequences from ITSa DGGE profiles were suitable for identification of toxic colonies. When information contained in ITSa is not sufficient then sequences covering the entire ITS (ITSc) confirmed the colony classification and identification of toxic groups based on ITSa and ITSc DGGE (sequences differed between classes and were mostly identical within a class). The occurrence of a few colonies within a class with slight (one basepair) sequence differences could be explained by the position of this basepair in the 3' region, which forms the high melting domain. In this region, base substitutions do not result in altered migration on DGGE. When positioned in the lower melting domain, one basepair differences could be detected clearly as evidenced by the upper and lower bands from e.g. colony #41.

Kurmayer et al. (13) analyzed subsamples of isolated colonies and reported contradictory mcyB gene detection in 5 out of 27 colonies. They explained this by either colony contamination by single cells, or by PCR inconsistencies. Our approach was not prone to PCR inconsistencies since identification of toxic strains was based on sequence information enclosed in a universal gene, instead of the detection of a specific gene using a diagnostic PCR. The information contained in the sequence was analyzed only after amplification. In contrast, in a diagnostic PCR, the amplification itself is a crucial step which is sensitive to variations in initial DNA concentration and quality and PCR conditions (notably the number of temperature cycles).

We detected more than one Microcystis sequences in 28% of the colonies we analyzed. Since the intensities of multiple bands in most profiles were similar, we considered it unlikely that additional bands originated from a few contaminating cells. It is unlikely that the ratio of target sequences altered significantly during PCR amplification, since all analyzed Microcystis sequences were very similar and primer sites were identical. Indeed, the ratio between initial cell numbers has been found to correlate well with DGGE band intensities after co-amplification of two Microcystis strains (data not shown). The occurrence of minor secondary bands in some profiles indicated that there was no overriding bias towards 1:1 mixtures of homologous PCR products as may be encountered in PCR amplification (24).

The multiple sequences in a portion of the colonies may be explained by contamination from aggregated colonies which were not separated despite thorough washings during isolation, or by the occurrence of multiple different rRNA operons in a portion of Microcystis strains. This can be resolved by further replating and cloning of the respective strains. The occurrence of different operons in a portion of the Microcystis strains would imply considerable intragenus variability. The most parsimonious explanation would be the presence in the genus Microcystis of at least two rRNA operons, identical in sequence in most strains and with mutations in one operon in a portion of the strains. The equal band intensities that were encountered in most colonies with multiple sequences are in agreement with the presence of two different operons in one strain. On the other hand, it could also imply that the different strains that comprised colonies were present in similar quantities. The alternative explanations for multiple colony bands may be true for different colonies. Some colonies may have consisted of aggregated strains as was supported by the presence of even three different sequences in colony #119, the presence of additional sequences of low intensity (e.g. Fig. 1, colony #70) and the occurrence of sequences as sole sequence in one colony and as one of two sequences in another colony (e.g. 146 and 147-u). Conversely, for some colonies the difference between bands could be well explained by a single basepair substitution (e.g. colonies #41, #47, #99, #139) or insertion/deletion at one location (#17) in one operon (of two present).

The detection of multiple sequences in a considerable portion of the carefully isolated and washed colonies, highlights that RFLP patterns or sequences obtained from such colonies (13) need to be interpreted with caution. A few isolated colonies (e.g. #78) probably contained a mixture of sequences even if they generated one band on ITSa and ITSc DGGE as judged from the many ambiguous base calls obtained from sequencing. Toxin data from these colonies can be assigned to sequences when the excised fragments are cloned. Unequivocal correlation of toxin production to one particular sequence was also not possible for the toxic colonies containing multiple sequences that could be separated by DGGE. Two sequences derived from a non-toxic colony will correspond to one or two non-toxic Microcystis strains, however, one of the sequences in a toxic colony could be derived from a non-toxic contaminant. There are two possible solutions to this problem of multiple bands in a toxic colony. The first solution would be to cultivate the cells from the colony of interest. Sequences and toxic properties of individual pure cultures obtained from these colonies will enable assigning toxic properties to the sequences of interest. A second solution would be based on expanding the available database of sequences assigned to toxic and non toxic strains to infer properties of the colonies of interest. The variation in Microcystis ITS between strains was restricted to approximately 11% of the sequence positions. The conserved regions contained elements necessary for coordinated transcription and processing of rRNA genes (8). Regions D1, D1', D2, D4, and D5 are required for correct folding of the rRNA transcript, indispensable for the prematuration of the 16S and 23S rRNA molecules by RNase III. Premature termination of transription of the rRNA operon is prevented by antiterminator sites. Antiterminator Box A sequences are reasonably well conserved within bacteria and are immediately preceded by a Box B stem of more variable sequence. Microcystis rRNA-ITS also contains one highly conserved tRNAile gene.

We did not find a relation between genotypes and origin of the isolated Microcystis colonies (Table 1). Although identical sequences were mostly shared between strains from the same lake, this could be explained by the small number of colonies representing most sequences. Sequences represented by multiple colonies originated from different locations (e.g. class 5a/11c). Elucidation of the occurrence of local strains would require a more comprehensive sampling of colonies in combination with analysis of DGGE profiles from different locations. DGGE analysis needs to be combined with sequencing as evidenced by e.g. excised bands e23, e25 and e29 which contained different sequences despite an apparently identical DGGE position.

There was also no relation between genotypic classification based on rRNA-ITS and morphospecies assignation. Every DGGE group with a considerable number of colonies contained representatives of different morphospecies (Table 1). This is in agreement with the difficulties inherent to morphological identification (see introduction), and the finding that different Microcystis morphotypes contain toxic and non-toxic strains (21, 29).

Microcystin concentration in water samples were well below the 10mg/L guideline for safe recreational waters (3) but exceeded this concentration considerably in scums. Although care is needed in quantitative interpretations of band intensities. The methods of the present invention are suitable for identification of the most abundant organisms in field populations. Initial cell numbers of Microcystis strains are likely to correlate well with DGGE band intensities since the efficiencies of DNA isolation and amplification are comparable (see also above). A non-toxic strain (bands e4, e5, e9, e21 and e22) was abundant in all samples from 't Joppe, and a strain of unknown toxicity (bands e2, e7, e13, e16 and e24) was present in June and August but had disappeared in September (Fig. 2 and Table 3).

Bands derived from toxic strains were detected only in the samples from June (bands e3 and e28) and not in the samples from August and September when Microcystis reached the highest densities (Table 2). In contrast, many non-toxic strains were identified in the profiles from August and September (e6, e8, e11, e12, e15, e17, e22, e25 and e29). In addition, most of the toxic colonies that had been isolated from these lakes (90%: 29 out of 32) originated from the June samples, and most of the non-toxic colonies (60%: 9 out of 13) from the August and September samples. Together, these data suggest that while in the beginning of a bloom toxic colonies dominated, they were outcompeted when the bloom progressed. This is in agreement with the negative correlation between microcystin content per cell and Microcystis abundance during a bloom that was found by Welker et al. (30). Some strains accumulated in scums as evidenced by the dominance of their bands in DGGE profiles of scum samples compared to water column samples from the same date (Fig 2). Their sequences corresponded to non-toxic strains (bands e12 and e25) or strains of unknown toxicity (bands e7, e10, e24 and e26). These data suggest that non-toxic strains accumulate in scums, perhaps because these strains have a greater capability for gas vesicle formation, or because toxic colonies sink out from the scums before non-toxic colonies.

Surprisingly, two dominant bands from the Zeegerplas profiles appeared to correspond to non-toxic colonies (bands e15= e29 and e17), while most isolated Zeegerplas colonies were found to contain microcystins. Two other dominant sequences (bands e13=e16 and e14) corresponded to colonies of unknown toxicity. Either during colony isolation or processing of the colonies, some bias must have occurred to explain this discrepancy. Also, the apparent abundance in many samples of (identical) bands e2, e7, e13 and e16 should have resulted in the isolation of the corresponding colonies. Possibly, this Microcystis strain forms small colonies as the isolation procedure was selective against small colonies (29). Alternatively, the dominant bands in community profiles could correspond to non-colony forming Microcystis strains that are missed by the colony isolation procedure yet are detected in community profiles generated from filtered water samples. A more intensive sampling and colony isolation study can be used to elucidate the sequence identity of all bands in the community profiles and thereby substantiate insights in the dynamics of Microcystis colonies.

### References example 2

1. Altschul, S. F., T. L. Madden, A. A. Schaffer, J. H. Zhang, Z. Zhang, W. Miller, and D. J. Lipman. 1997. Gapped BLAST and PSI-BLAST: a new generation of protein database search programs. Nucleic Acids Res. 25:3389-3402.
2. Bolch, C. J. S., S. I. Blackburn, G. J. Jones, P. T. Orr, and P. M. Grewe. 1997. Plasmid content and distribution in the cyanobacterial genus Microcystis Kützing ex Lemmermann (cyanobacteria: Chroococcales). Phycologia 36:6-11.
3. Chorus, I., and J. Bartram. 1999. Toxic cyanobacteria in water. E & FN Spon, London.
4. Chorus, I., V. Niesel, J. Fastner, C. Wiedner, B. Nixdorf, and K.-E. Linden-schmidt. 2001. Environmental factors and microcystin levels in water bodies, p. 159-177. In I. Chorus (ed.), Cyanotoxins: occurrences, causes, consequences. Springer-Verlag KG, Berlin, Germany.
5. Dittmann, E., B. A. Neilan, M. Erhard, H. von Döhren, and T. Börner. 1997. Insertional mutagenesis of a peptide synthetase gene that is responsible for hepatotoxin production in the cyanobacterium Microcystis aeruginosa PCC 7806. Mol. Microbiol. 26:779-787.
6. Fastner, J., M. Erhard, and H. von Döhren. 2001. Determination of oligopeptide diversity within a natural population of Microcystis spp. (Cyanobacteria) by typing single colonies by matrix-assisted laser desorption ionization-time of flight mass spectrometry. Appl. Environ. Microbiol. 67:5069-5076.
7. Hall, T. A. 1999. BioEdit: a user-friendly biological sequence alignment editor and analysis program for Windows 95/98/NT. Nucl. Acids. Symp. Ser. 41:95-98.
8. Iteman, I., R. Rippka, N. Tandeau de Marsac, and M. Herdman. 2000. Comparison of conserved structural and regulatory domains within divergent 16S rRNA-23S rRNA spacer sequences of cyanobacteria. Microbiology 146:1275-1286.
9. Janse, I., M. Meima, W. E. A. Kardinaal, and G. Zwart. 2003. High resolution differentiation of cyanobacteria using rRNA-ITS DGGE. Appl. Environ. Microbiol.
10. Kaebernick, M., T. Rohrlack, K. Christoffersen, and B. A. Neilan. 2001. A spontaneous mutant of microcystin biosynthesis: genetic characterization and effect on Daphnia. Environ. Microbiol. 3:669-679.
11. Komárek, J., and K. Anagnostidis. 1999. Cyanoprokaryota, 1. Teil Chroococcales, p. 225-236. Gustav Fischer Verlag, Jena.
12. Kurmayer, R., G. Christiansen, and I. Chorus. 2003. The abundance of microcystin-producing genotypes correlates positively with colony size in Microcystis sp and determines its microcystin net production in Lake Wannsee. Appl. Environ. Microbiol. 69:787-795.
13. Kurmayer, R., E. Dittmann, J. Fastner, and I. Chorus. 2002. Diversity of microcystin genes within a population of the toxic cyanobacterium Microcystis spp. in Lake Wannsee (Berlin, Germany). Microb. Ecol. 43:107-118.
14. Long, B. M., G. J. Jones, and P. T. Orr. 2001. Cellular microcystin content in N-limited Microcystis aeruginosa can be predicted from growth rate. Appl. Environ. Microbiol. 67:278-283.
15. Mikalsen, B., G. Boison, O. M. Skulberg, J. Fastner, W. Davies, T. M. Gabrielsen, K. Rudi, and K. S. Jakobsen. 2003. Natural variation in the microcystin synthetase operon mcyABC and impact on microcystin production in Microcystis strains. J. Bacteriol. 185:2774-2785.
16. Muyzer, G., E. C. de Waal, and A. G. Uitterlinden. 1993. Profiling of complex microbial populations by denaturing gradient gel electrophoresis analysis of polymerase chain reaction-amplified genes coding for 16S rRNA. Appl. Environ. Microbiol. 59:695-700.
17. Neilan, B. A., E. Dittmann, L. Rouhiainen, R. A. Bass, V. Schaub, K. Sivonen, and T. Börner. 1999. Nonribosomal peptide synthesis and toxigenicity of cyanobacteria. J. Bacteriol. 181:4089.
18. Nishizawa, T., M. Asayama, K. Fujii, K. Harada, and M. Shirai. 1999. Genetic analysis of the peptide synthetase genes for a cyclic heptapeptide microcystin in Microcystis spp. J. Biochem. (Tokyo) 126:520.
19. Orr, P. T., and G. J. Jones. 1998. Relationship between microcystin production and cell division rates in nitrogen-limited Microcystis aeruginosa cultures. Limnology and Oceanography 43:1604-1614.
20. Otsuka, S., S. Suda, R. H. Li, S. Matsumoto, and M. M. Watanabe. 2000. Morphological variability of colonies of Microcystis morphospecies in culture. J. Gen. Appl. Microbiol. 46:39-50.
21. Otsuka, S., S. Suda, R. H. Li, M. Watanabe, H. Oyaizu, S. Matsumoto, and M. M. Watanabe. 1999. Phylogenetic relationships between toxic and non-toxic strains of the genus Microcystis based on 16S to 23S internal transcribed spacer sequence. FEMS Microbiol. Lett. 172:15-21.
22. Rippka, R. 1988. Isolation and purification of cyanobacteria. Meth. Enzymol. 167:3-27.
23. Sivonen, K., and G. J. Jones. 1999. Cyanobacterial toxins, p. 41-111. In I. Chorus and J. Bartram (ed.), Toxic cyanobacteria in water. E. & F. N. Spon, London.
24. Suzuki, M. T., and S. J. Giovannoni. 1996. Bias caused by template annealing in the amplification of mixtures of 16S rRNA genes by PCR. Appl. Environ. Microbiol. 62:625-630.
25. Tillett, D., E. Dittmann, M. Erhard, H. von Dohren, T. Borner, and B. A. Neilan. 2000. Structural organization of microcystin biosynthesis in Microcystis aeruginosa PCC7806: an integrated peptide- polyketide synthetase system. Chem. Biol. 7:753-764.
26. Tillett, D., D. L. Parker, and B. A. Neilan. 2001. Detection of toxigenicity by a probe for the microcystin synthetase A gene (mcyA) of the cyanobacterial genus Microcystis, comparison of toxicities with 16S rRNA and phycocyanin operon (phycocyanin intergenic spacer) phylogenies. Appl. Environ. Microbiol. 67:2810-2818.
27. Van de Peer, Y., and R. De Wachter. 1994. TREECON for Windows: a software package for the construction and drawing of evolutionary trees for the Microsoft Windows environment. Comput. Applic. Biosci 10:569-570.
28. Vezie, C., L. Brient, K. Sivonen, G. Bertru, J. C. Lefeuvre, and M. Salkinoja-Salonen. 1998. Variation of microcystin content of cyanobacterial blooms and isolated strains in Lake Grand-Lieu (France). Microb. Ecol. 35:126-135.
29. Via-Ordorika, L., J. Fastner, M. Hisbergues, E. Dittmann, M. Erhard, J. Komarek, R. Kurmayer, and I. Chorus. 2004. Distribution of microcystin-producing and non-producing Microcystis in European freshwater bodies: detection of microcystins and mcy genes in single colonies. in prep.
30. Welker, M., H. von Dohren, H. Tauscher, C. E. W. Steinberg, and M. Erhard. 2003. Toxic Microcystis in shallow lake Müggelsee (Germany) - dynamics, distribution, diversity. Arch. Hydrobiol. 157:227-248.
31. Zwart, G., W. D. Hiorns, B. A. Methe, M. P. Van Agterveld, R. Huismans, S. C. Nold, J. P. Zehr, and H. J. Laanbroek. 1998. Nearly identical 16S rRNA sequences recovered from lakes in North America and Europe indicate the existence of clades of globally distributed freshwater bacteria. Syst. Appl. Microbiol. 21:546-556.

### Figure legends of example 2

Fig.1. Example of an ITSa DGGE of 15 isolated colonies. Each unique gel position was assigned a number (see table 1), e.g. numbers 10 and 17 for colony 70; number 1 for colonies 69 , 65 and 21, number 24 for colony 68. h= heteroduplex.
Fig. 2. Cyanobacterial community composition of lakewater samples analyzed by ITSa (left gel) and ITSc (right gel) DGGE. Samples were taken from lakes 't Joppe and Zeegerplas in June, August and September 2001. An S following the sampling date signifies scum samples. Bands that yielded useable sequences after excision, reamplification and sequencing are numbered. Information on these sequences is presented in Table 3.

Reversed Line Blot showing hybridization of the probes designed for specific detection of groups of toxic and non-toxic Microcystis strains.
Increasing concentrations (0.66, 2, 6.6, 20 mM from left to right) of probes P1a354(52), Plall, P2all and P1514 were bound to the membrane in parallel vertical lanes. The rRNA-ITS from a number of colonies of known toxin production was PCR amplified, hybridized to these probes in horizontal lanes, and washed. Colonies are indicated with their identification number (BK#), followed by a symbol indicating whether they are known to produce toxins (T= toxic, NT = non-toxic) and a group number. Group numbers had been assigned to colonies based on the clustering of their rRNA-ITS sequences.
Probe P1514 was designed as general probe to hybridize with all Microcystis strains and thus serve as positive hybridization control. All sequences tested hybridized with this probe. Probe Pla354 (52) was designed to hybridize with strain 1A only (which includes only toxic isolates). Only group 1A sequences hybridized with this probe, no cross-reactivity was observed. Probe P1all was designed to hybridize with all strains from cluster 1 (which includes toxic strains 1A and 1B and non-toxic strains 1C and 1D). 1A, 1B and 1C sequences hybridized with this probe, however, one sequence from strain7 (BK10-o) also did, which call for further optimization of reaction conditions. Probe P2all was designed to hybridize with all strains from cluster 2 (which includes toxic strains 2A and 2B). Only strain 2A and 2B sequences hybridized with this probe and no cross-reactivity was observed.

### Example 3

### MATERIALS AND METHODS Example 3

### Probe design

Probes were designed on the basis of rRNA ITS sequences from 129 different cyanobacterial isolates (EMBL database accession numbers AB015357 to AB015403 {Otsuka et al, 1999} and AJ605140 to AJ605221. The sequences were aligned using the software ARB to the alignment/database, release june 2002, provided by W. Ludwig of the ARB bioinformatics group (http://www2.mikro.biologie.tu-muenchen.de/arb/). Using the program Treecon (van de Peer et al) a distance tree was calculated using a neighbor joining algorithm. Among the 129 ITS sequences we identified 13 clusters of sequences. Within each cluster, the cluster members (sequences) were relatively similar in comparison to sequences outside of the cluster. Cluster 1 was subdivided into 4 strains (strain 1a,1b,1c and 1d), cluster2 was subdivided into 2 strains (strain 2a and 2b) and cluster 5 was subdivided into 6 strains (strain 5a, 5b, 5c, 5d, 5e and 5f). All other clusters (3,4,6 to 13) were designated strains for the purpose of the present invention. For each strain, sequences belonging to this strain had very high similarity or were identical. For each strain and each cluster, multiple probes were designed, 20 nucleotides in length, using the tool 'probe design' in the package ARB. Using the tool 'probe match' a first evaluation of the probes was made in which we selected probes that had perfect match (full complementarity) to all sequences in of the strain or cluster and 2 or more nucleotide mismatches to any sequence outside of the strain or cluster. If such a probe could not be designed for a particular strain or cluster, we selected a probe that had a single mismatch to all sequences outside of the strain or cluster. In addition probes were found that were specific for a combination of strains such for strains 8 and 9, for strains 2b, 5a and 6, for strains 2b 5d 5e and 6, for strains 10, 11, 12, 13 (Table 3B of Example 3)
The output of probe match, which is a list of matching sequences to the investigated probe, also contains flanking sequences. If possible, we extended each probe with 1 to 8 flanking nucleotides (adjacent to the probe sequence at 3' and 5' side) that were shared by all sequences that had perfect match to the 20 nucleotide investigated probe.
These extended probe sequences were subjected to a second round of probe design using the software Array Designer (Premier Biosoft International, Palo Alto, CA, USA) in order to generate probes of similar theoretical melting temperatures (Tm). The package selects a contiguous stretch of nucleotides from each input sequence (the extended probe sequences) to best approach a chosen target Tm as calculated using nearest neighbor thermodynamic theory with SantaLucia thermodynamics values and to best agree to a number of additional probe settings. These additional probe design settings were as follows: The maximum acceptable free energy of hairpin formation was set at 6.0 -kcal/mol; the maximum acceptable free energy for self dimer formation was set at 12.0 -kcal/mol and the maximum acceptable run repeat length of nucleotides and dinucleotides was set at 8. If length of the input sequence and settings allowed, for each extended probe sequence, three probes were designed: one with theoretical Tm of 52°C (± 1°C), one with theoretical Tm of 50°C (± 1°C), and one with theoretical Tm of 48°C (± 1°C). The selected probes (Table 3A and B of Example 3) had lengths of 17 to 28 nucleotides.
For each probe that had a single mismatch to the closest sequence outside of the target strain or cluster, we also designed a counterpart probe that contained the mismatching nucleotide that was shared by the majority of the closest sequence outside of this strain or cluster. The remaining nucleotides of this 1-mismatch counterpart probe were identical to the perfectly matching probe, but, if necessary one or two, perfectly matching, extra nucleotides were added at the 3' side and/or 5'side. This addition was deemed necessary if the mismatch was an A or T to replace a G or C. In this manner the counterpart mismatch probe could be designed such that the theoretical melting temperature was identical to or slightly higher than that of the theoretical melting temperature of the perfectly matching probe.

### Reverse line blot hybridization.

In reverse blot hybridization, probes are covalently bound to a membrane and the target DNA is applied in solution {Dattagupta, 1989; Saiki, 1989}. Reverse line blot hybridization is a modification introduced by Kaufhold et al {Kaufhold,1994} in which the probes are applied as lines instead of dots. Hybridization with labeled target DNA in lines perpendicular to the probe lines enables simultaneous testing of several samples to several probes. A detailed protocol is available at http://www.nioo.knaw.nl/cl/me/.

Membrane preparation. For covalent linkage of probes, a Biodyne C membrane (Pall Europe Ltd, Portsmouth, UK) is activated by incubation for 10 min in a rolling bottle at room temperature in 16% 1-ethyl-3-(3-dimethylaminopropyl)carbodiimide (Sigma-Aldrich, St. Louis, MO), rinsed with tapwater, and placed on a PC200 support cushion (Immunetics, Cambridge, MA) in a Miniblotter 45 manifold (Immunetics), with screws handtight. Slots are filled with 150 µl of 0.66, 2, 6.6, 20 µM probe solution (probes in Table 3 Example 3) in 500 mM NaHCO3 (pH 8.4). Probes are allowed to bind at room temperature for 5 minutes, after which the probe solution is aspirated from the slots. The blot is then removed from the manifold using forceps and incubated for 8 minutes at room temperature in 100 mM NaOH in a rolling bottle to inactivate the membrane. Then, the membrane is shaken gently, in 200 ml 2 x SSPE/0.1% sodium dodecyl sulphate (SDS) at 60°C for 5 min (SSPE = 18 mM NaCl, 1 mM NaH2PO4, 0.1 mM NaEDTA, pH 7.4). For storage, the prepared membrane is washed in 200 ml 20 mM NaEDTA pH 8 at room temperature for 15 min, sealed in seal foil (Audion Electro, Weesp, Netherlands) to avoid dehydration and kept at 4°C until use.

Hybridization. After incubation in 2 x SSPE/0.1% SDS at room temperature for 5 min, the probe-carrying membrane is placed on the support cushion in the manifold, rotated 90° from the orientation of probe application. Each PCR products is denatured in 150 µl 2 x SSPE/0.1% SDS in a 0.5 ml microcentrifuge tube at 99°C for 10 min and immediately thereafter chilled on ice. Then, all PCR products are applied to the slots and slots without PCR product are filled with 2 x SSPE/0.1% SDS. Hybridization is performed in the manifold at 42°C for 60 min on a horizontal surface without agitation. After the hybridization, the membrane is washed by two subsequent incubations in a rolling bottle (with mesh) in 200 ml 2 x SSPE/0.5% SDS at 52°C for 10 min (stringent wash). Detection. In the next step, the washed membrane is incubated in 10 ml of a 1:4000 dilution of peroxidase labeled streptavidin conjugate (Roche, Mannheim, Germany) in 2 x SSPE/0.5% SDS, in a rolling bottle at 42°C, for 45 min. The membrane is then washed twice in 200 ml 2 x SSPE/0.5% SDS at 42°C for 10 min and rinsed twice in 200 ml 2x SSPE at room temperature for 5 min. After incubation in 10 ml BM Chemiluminescence Blotting Substrate (POD) (Roche) for 1 min the membrane is covered with an overhead sheet and X-ray film for detection of chemiluminescence. The film is exposed 30 minutes for PCR products from control templates (16S rRNA gene clones) or overnight for natural samples. Signal strengths (pixel volumes) are recorded from the X-ray film using a CCD camera and analyzed using the software package Phoretics 1D Advanced (Non-linear Dynamics, UK).

### Results

### Probe design,

On the basis of an optimized alignment and phylogenetic analysis of ITS sequences from 129 strains, we designed 94 probes specific for particular strains of *Microcystis* or clusters of strains thereof (Table 3A and B of Example 3 and Fig. 1 of Example 3). For this we followed the design strategy as outlined in the materials and methods section. When possible for each strain and cluster and each target site (in which the target site is the small region of 12 to 30 nucleotides that shows differences between clusters or strains), 3 probes were designed, differering from each other by only a few nucleotides. These three probes were designed to have theoretical melting temperatures of approximately 48, 50 and 52 °C. Each of these variant probes has different melting characteristics when tested in actual hybridization, thus allowing selection of the optimal probe for particular assays. The optimal probe pairs high sensitivity with high selectivity.
13 probes were selected that were highly specific, meaning that they have perfect match for sequences of the target strain or cluster and two or more mismatches to strains that are not part of the target strain or cluster. For probe P2all252-48 longer versions of the probe, with higher theoretical melting temperatures could not be designed because of the variation of the flanking nucleotides within cluster 2.
33 probes were selected that had perfect match to most sequences from the target strain or cluster and one or more mismatches to sequences that are not part of the target strain or cluster. For each of these probes we designed a counterpart probe of similar theoretical melting temperature that has the same target site as the specific probe but differs at the nucleotide that most commonly mismatched to the ITS sequences with a single mismatch. Testing of a sample against both the perfect matching and the mismatching counterpart probe will increase the certainty of classification of the test cyanobacterium.

### Hybridization in Reversed Line Blot.

The designed probes were tested in Reversed Line Blot hybridization. Fig. 2 of example 3 shows an example of a hybridization result.
We used DNA originating from isolated Microcystis colonies as test DNA. The isolated colonies used for this example were K63-b, K15-o, K60, K12, K65. K13, K21, K75-b, K69, K75-o, K10-o, K51, K72, K123, K10-b, K104, K139-o, K47-o, K74, K57, K34, K58, K16, K49. These colonies were obtained from lake water samples through microscope assisted micromanipulation as described in example 2. After DNA isolation from these colonies and specific amplification of the ITSc region as described in Example 2, the amplified DNA was subjected to DGGE and bands were excised from the gel, as described in Example 2. The test DNA was obtained through amplification using the ITSc primers of the DNA extracted from these excised bands as described in Example 2. This amplified known test DNA was then subjected to hybridization in Reverse Line Blot.
Probe P1514 -52 was designed as general probe to hybridize with all *Microcystis* strains and thus serves as positive hybridization control. All sequences tested hybridized with this probe. Probe P1a251 -52 was designed to hybridize with group 1A only (which includes only toxic strains). Only group 1A sequences hybridized with this probe, no cross-reactivity was observed (Figure 2 of Example 3). Probe P1all36 -52 was designed to hybridize with all strains from cluster 1 (which includes toxic strains 1A and 1B and non-toxic strains 1C and 1D). 1A, 1B and 1C sequences hybridized with this probe, however, one sequence from strain 6 (K10-o) also did (Figure 2 of Example3). Probe P2all252 -52 was designed to hybridize with all strains from cluster 2 (which includes toxic strains 2A and 2B). Only strain 2A and 2B sequences hybridized with this probe and no cross-reactivity was observed (Figure 2 of Example3).

### References example 3:

Kaufhold, A., A. Podbielski, G. Baumgarten, M. Blokpoel, J. Top, and L. Schouls. 1994. Rapid typing of group-a streptococci by the use of DNA amplification and nonradioactive allele-specific oligonucleotide probes. FEMS Microbiol. Lett. 119:19-25.
Otsuka, S., S. Suda, R. H. Li, M. Watanabe, H. Oyaizu, S. Matsumoto, and M. M. Watanabe. 1999. Phylogenetic relationships between toxic and non-toxic strains of the genus Microcystis based on 16S to 23S internal transcribed spacer sequence. FEMS Microbiol. Lett. 172:15-21.
Saiki, R. K., P. S. Walsh, C. H. Levenson, and H. A. Erlich. 1989. Genetic analysis of amplified DNA with immobilized sequence- specific oligonucleotide probes. Proc. Natl. Acad. Sci. U. S. A. 86:6230-6234.
Dattagupta, N., P. M. M. Rae, E. D. Huguenel, E. Carlson, A. Lyga, J. A. Shapiro, and J. P. Albarella. 1989. Rapid identification of microorganisms by nucleic-acid hybridization after labeling the test sample. Anal. Biochem. 177:85-89.

**TABLE 1.**

| *EXAMPLE 1 Cultures of cyanobacteria used in this study* | | | |
|---|---|---|---|
| **Code** | **Identity** | **Origin** | **Reference**^{a} |
| K29, K50 | *Microcystis* sp. | Kinselmeer, The Netherlands | This study |
| S2 | *Microcystis* sp. | Sneekermeer, The Netherlands | This study |
| V28, V40, V67, V72, V73, V80, V88, V89, V91 | *Microcystis* sp. | Volkerak, The Netherlands | This study |
| Z6, Z11 | *Microcystis* sp. | Zeegerplas, The Netherlands | This study |
| CYA 43 | *Microcystis aeruginosa* Kütz | USA | NIVA |
| CYA 140 | *Microcystis aeruginosa* Kütz | Bendig's Pond, Bruno, Canada | NIVA |
| CYA 228 | *Microcystis aeruginosa* Kütz | Lake Akersvatnet, Vestfold, Norway | NIVA |
| PCC 7806 | *Microcystis aeruginosa* | Braakman Reservoir, The Netherlands | PCC |
| PCC 7820 | *Microcystis aeruginosa* | Loch Balgavies, Scotland | PCC |
| SAG17.85 | *Microcystis aeruginosa* Kütz | Lake Neusiedler, Austria | SAG |
| PCC 6803 | *Synechocystis* sp. | Freshwater, California, USA | PCC |
| PCC 7942 | *Synechococcus* sp. | Freshwater, California, USA | PCC |
| PCC 73110 | *Leptolyngbya* sp. | | PCC |
| CYA 99 | *Lyngbya* sp. | River Suldalslågen | NIVA |
| | *Microcoleus* sp. | Microbial mat from hypersaline Lake Chirana, Spain | (16) |
| CYA 146 | *Pseudanabaena catenata* Lauterb. | Canada | NIVA |
| CYA 24 | *Planktothrix prolifica* | Lake Levrasjön, Skäne, Sweden | NIVA |
| CYA 126 | *Planktothrix aghardii* | Lake Långajön, Finland | NIVA |
| ATCC 29413 | *Anabaena variablis* | | ATCC |
| CYA 135 | *Anaboenopisis arnoldii* Aptek. | Ferguson Gulf, Lake Turkana, Kenya | NIVA |
| CYA 103 | *Aphanizomenon flos-aquae* f. *gracile* | Pond at Vingrom, Lillehammer, Norway | NIVA |
| | (Lemm.) | | |
| 1401/7 CCAP | *Aphanizomenon flos-aquae* | Freshwater, Brantry Lough, N. Ireland | CCAP |
| CYA 225 | *Cylindrospermapsis raciborskii* | Lake Balaton, Hungary | NIVA |
| CYA 124 | *Nostoc* sp. | Lake Steinsfjorden, Buskerud, Norway | NIVA |
| KAC 13 | *Nodularia spumigena* | Baltic Sea | KAC |
| BV16R | *Gloeotrichia* sp. | Lake Broekvelden, The Netherlands | This study |
| IMS 101 | *Trichodesmium erythraeum* | North Carolina coast | KAC |
| PCC 9006 | *Prochlorothrix hollandica* | Lake Loosdrecht, The Netherlands | PCC |

| | | | |
|---|---|---|---|
| *a* NIVA: Culture Collection of Algae, Norwegian Institute for Water Research; PCC: Pasteur Culture Collection of Cyanobacteria, Institut Pasteur, Paris, France; KAC: Kalmar Algae Collections, Kalmar University, Sweden; SAG: Culture Collection of Algae at the University of Göttingen, Germany; CCAP: Culture Collection of Algae and Protozoa, CEH, Windermere, UK; ATCC: American Type Culture Collection, Manassas, Virginia, USA. | | | |

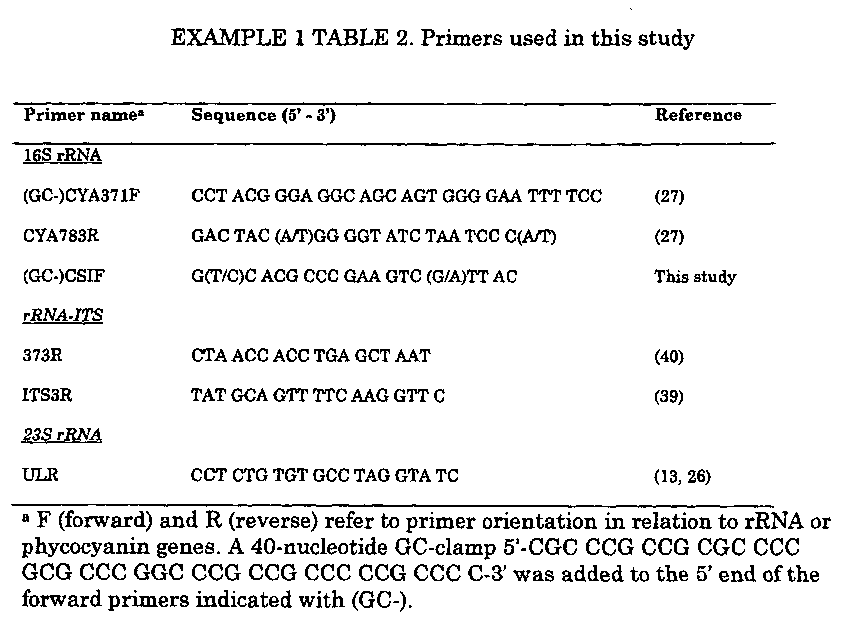

**TABLE 3.**

| EXAMPLE 1 Number and approximate sizes of the PCR products of strains from various cyanobacterial genera amplified with the primers described in this study | | | |
|---|---|---|---|
| Strain | ITSa^{a} | ITSb^{a} | ITSc^{a} |
| Microcystis | 300 | 500 | 550 |
| aeruginosa | | | |
| *Synechocystis* sp. | 325 | | 675 |
| *Synechococcus* | 325 | 650 | 750 |
| *Leptolyngbya* | 275 | | 500 |
| *Lyngbya* sp. | 300 | | 675 |
| *Microcoleus* sp. | 300 | n.d. | 675 |
| *Pseudanabaena catenata* | 300 | 675 | 750 |
| *Planktothrix prolifica* | 300 | 550 | 525, 700 |
| *Planktothrix aghardi* | 300 | 550 | 525, 700 |
| *Anabaena variabilis* | 300 | 375, 600 | 500, 700 |
| *Anabaenopsis arnoldii.* | 300 | 350, 650 | 450, 725 |
| *Aphanizomenon flos-aquae* | 300 | 400, 625 | 475, 700 |
| *Aphanizomenon flos-aquae* f. *gracile* | 300 | 400, 625 | 475, 700 |
| *Cylindrospermopsis raciborskii* | 300 | 350, 525 | 425, 600 |
| *Nostoc* sp. | 300 | 375, 650 | 500, 775 |
| *Nodularia spumigena* | 300 | 400, 700 | 575, 875 |
| *Gloeotrichia* sp. | 275 | 375, 675 | 475, 775 |
| *Trichodesmium erythraeum* | 300 | 550 | 675 |
| *Prochlorothrix hollandica* | 350 | 800 | 1050 |

| | | | |
|---|---|---|---|
| ^{a} Primer combinations are described in the text, the forward primers include a 40 nucleotide GC-clamp. PCR product sizes (in basepairs) were estimated from agarose gels; n.d. means not determined | | | |

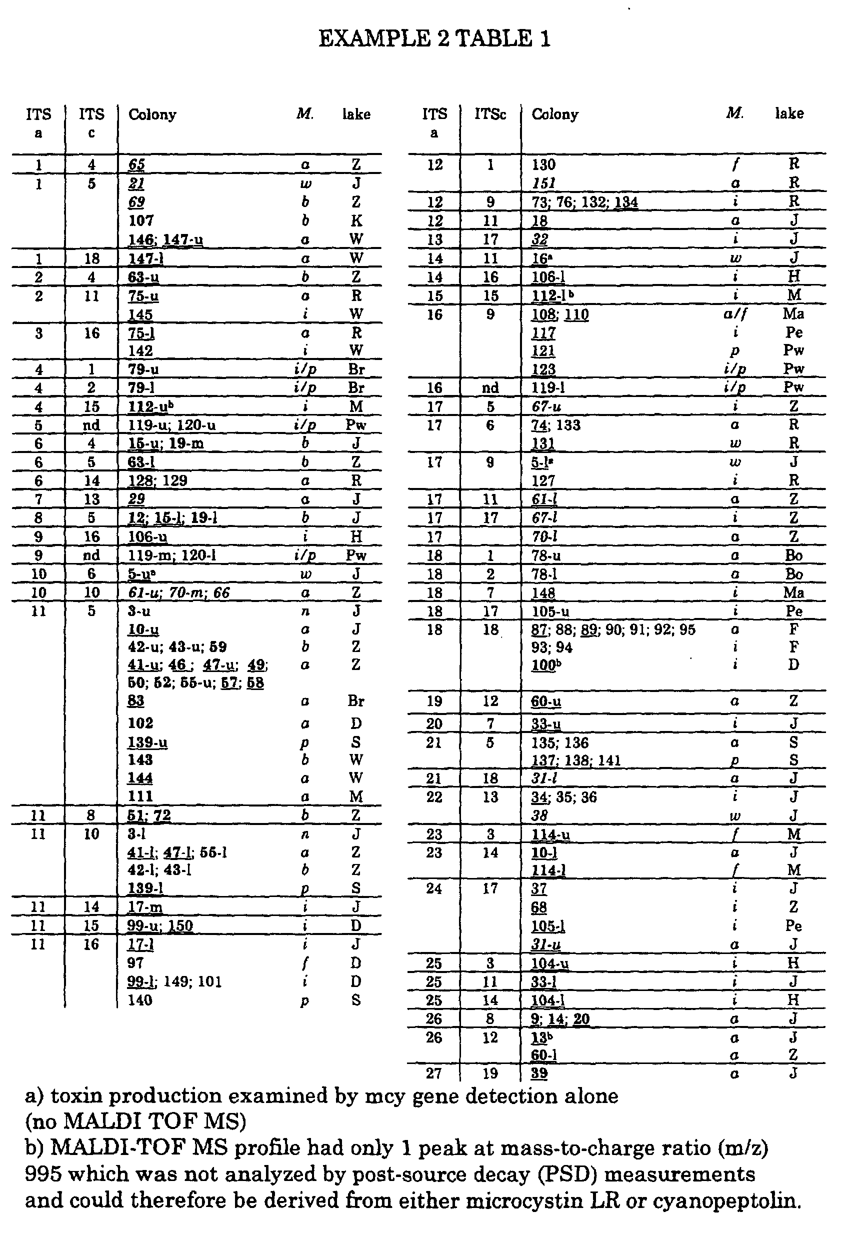

Table 1: Grouping of isolated Microcystis colonies based on rRNA-ITS DGGE.
Colonies were analyzed using ITSa and ITSc DGGE and assigned group
numbers based on the positions of their bands. Morphospecies assignment and
lake of origin are given for each colony. Colonies in bold are toxic as was
determined by MALDI-TOF or in a few cases by amplification of the mcy gene.
Toxicity data are lacking for colonies printed in italics. Underlined bands were
sequenced. The suffix –u refers to the upper band in the ITSc DGGE profile
from a colony, the suffix –m refers to the middle band, and suffix -l refers to
the lower band. If the upper and lower band from a colony have the same
group assignment for e.g. ITSa, this means that there is one band in the ITSa
profile, and two in the ITSc profile. Note that if a mixture of 2 sequences gave
rise to 2 ITSa and ITSc bands, the relative band positions (meaning upper or
lower) may be reversed. M.= Microcystis morphospecies: a= aeruginosa; b=
botrys; i= ichthyoblabe; f= flos-aquae; n= novacekii; p= panniformis; w=
wesenbergii. Lakes: Br= Brno, Czechia; Bo= Bordeaux, France; D= Dundee,
Scotland; F= lake Frederikborg, Denmark; H= Holzöstersee, Austria; J= 't
Joppe, the Netherlands; K= Krossin, E ?; W= Wannsee, Germany; M=
Mügelsee, Germany; Ma= E ?, Marocco; Pw= E pw?, Portugal; Pe= E: Pe?,
Portugal; R= Rescoby, Scotland; S= E: Sicilia ?; Z= Zeegerplas, the
Netherlands.

**TABLE 2**

| EXAMPLE 2 Cell counts and ambient environmental parameters during sampling for *Microcystis* colonies | | | | | | | |
|---|---|---|---|---|---|---|---|
| sampling | | | cell counts | | | toxins | |
| lake | date | source | *Microcystis* | *Anabaena* | *Aphanizo menon* | Microcystin in water column (µg/L) | Microcystin per cell (pg/cell) |
| 't Jopp e | 27.06 | water column | 8,1 × 10³ | 2,4 × 10⁴ | 4,5 × 10² | 0.22 (0.18) | 27 |
| 't Jopp e | 29.08 | water column | 5,5 × 10⁴ | 5,3 × 10² | 0 | 2.64 (0.03) | 48 |
| 't Jopp e | 29.08 | scum | 5,6 × 10⁶ | n.d. | n.d. | 420 (22.9) | 75 |
| 't Jopp e | 28.09 | water column | 7,8 × 10⁴ | 1,4 × 10³ | 88 | 0 | 0 |
| 't Jopp e | 28.09 | scum | 6,1 × 10⁷ | n.d. | n.d. | 3308 (244) | 55 |
| Zeeg erpla a | 20.06 | water column | 5,4 × 10⁴ | 1,8 × 10⁵ | 0 | 0 | 0 |
| Zeeg erpla s | 20.08 | water column | 2,8 × 10⁵ | 1,0 × 10⁵ | 0 | 0.60 (0.09) | 2 |

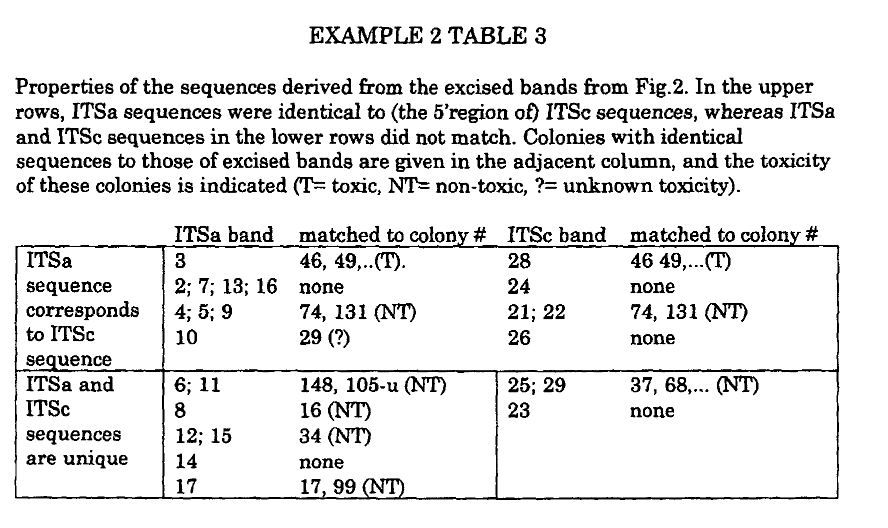

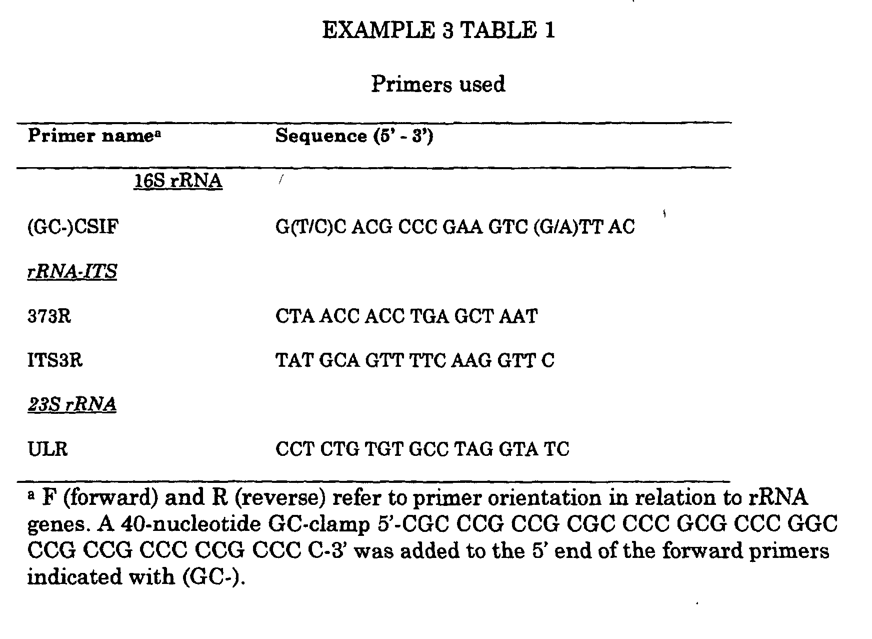

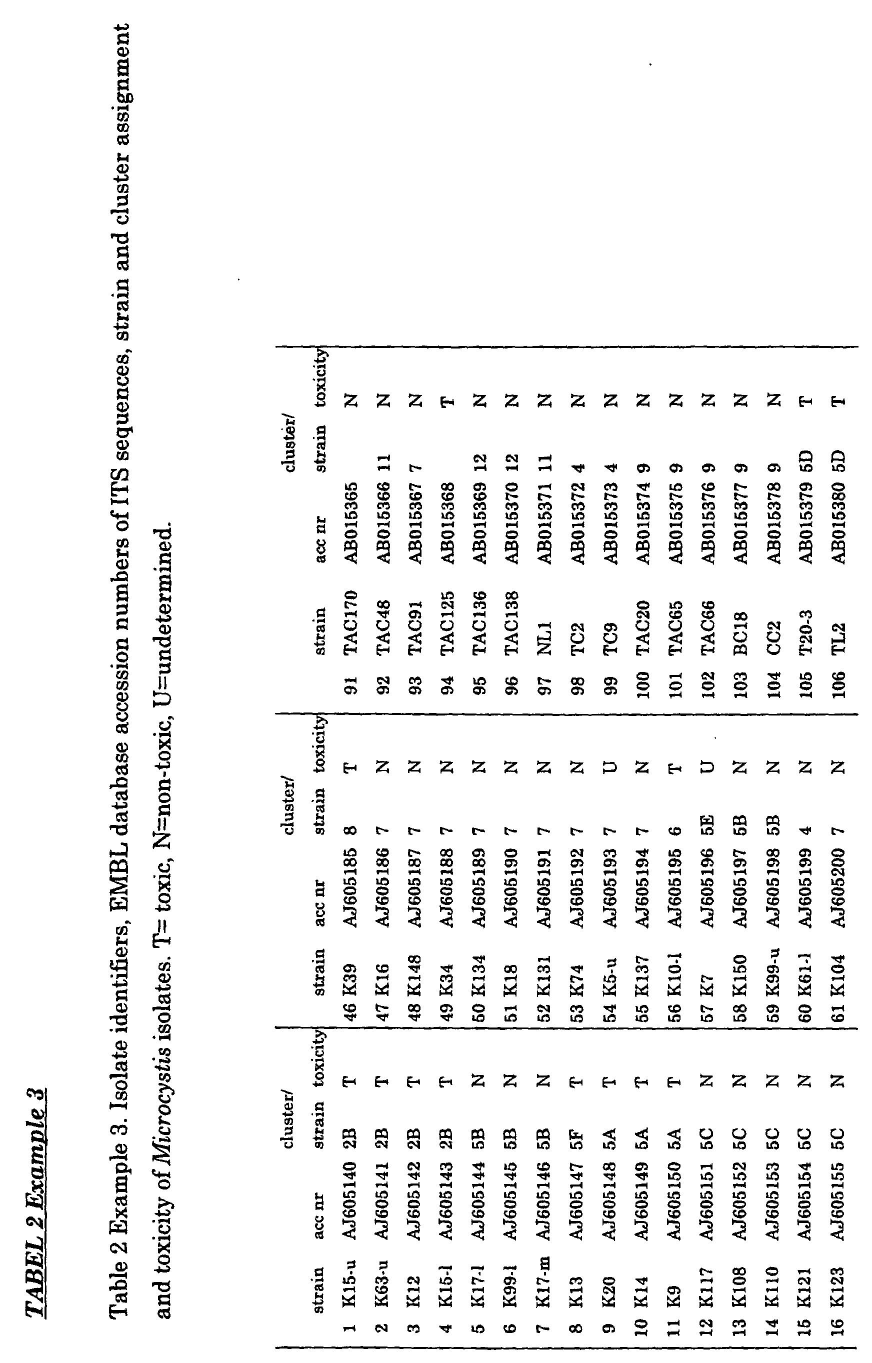

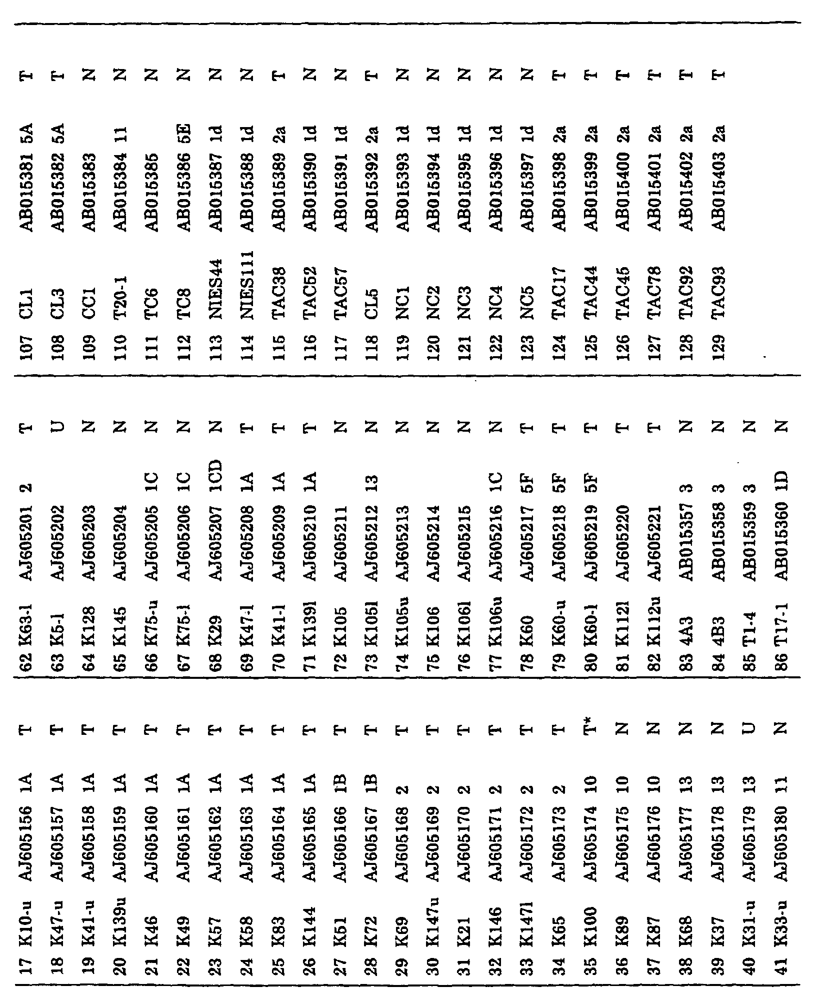

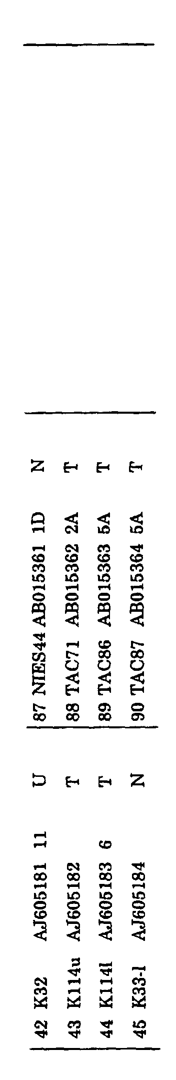

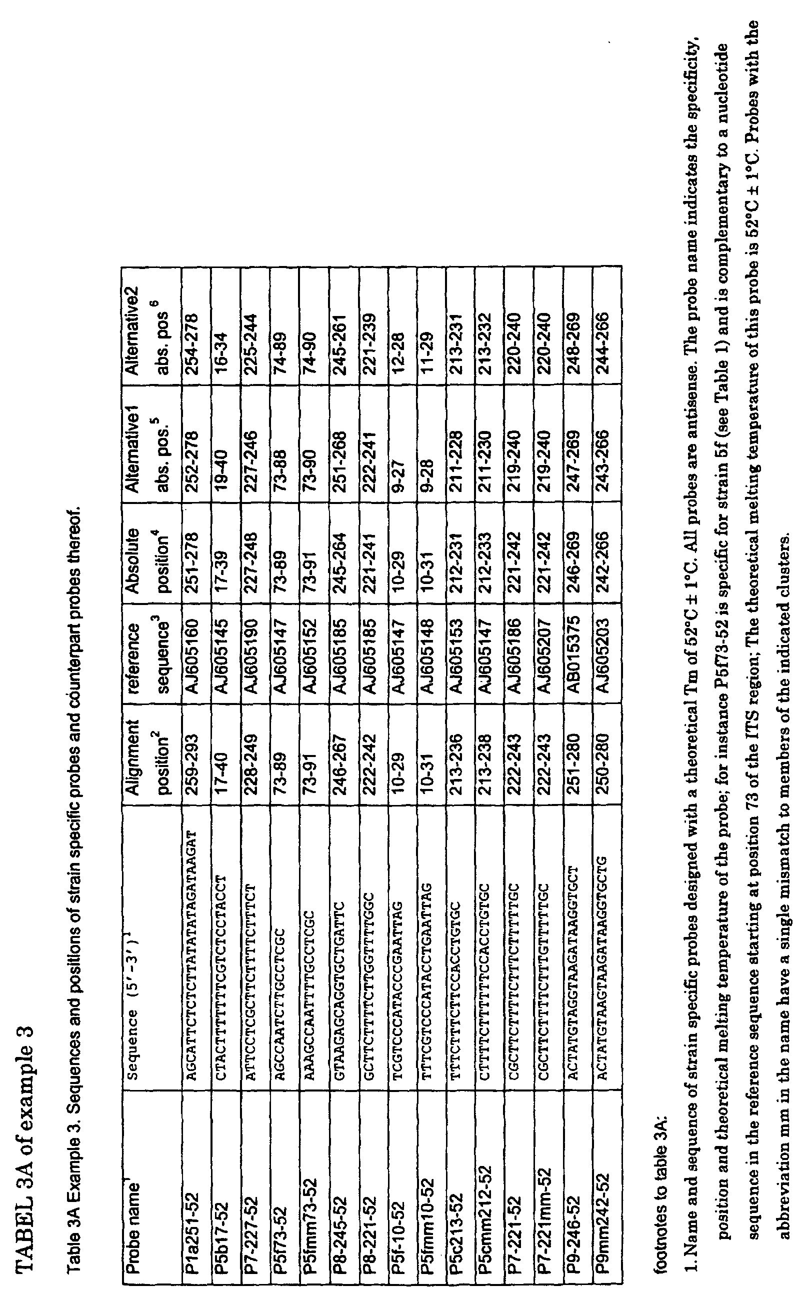

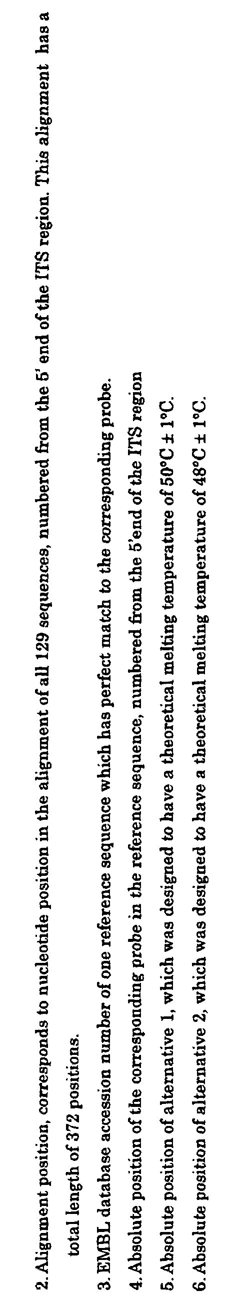

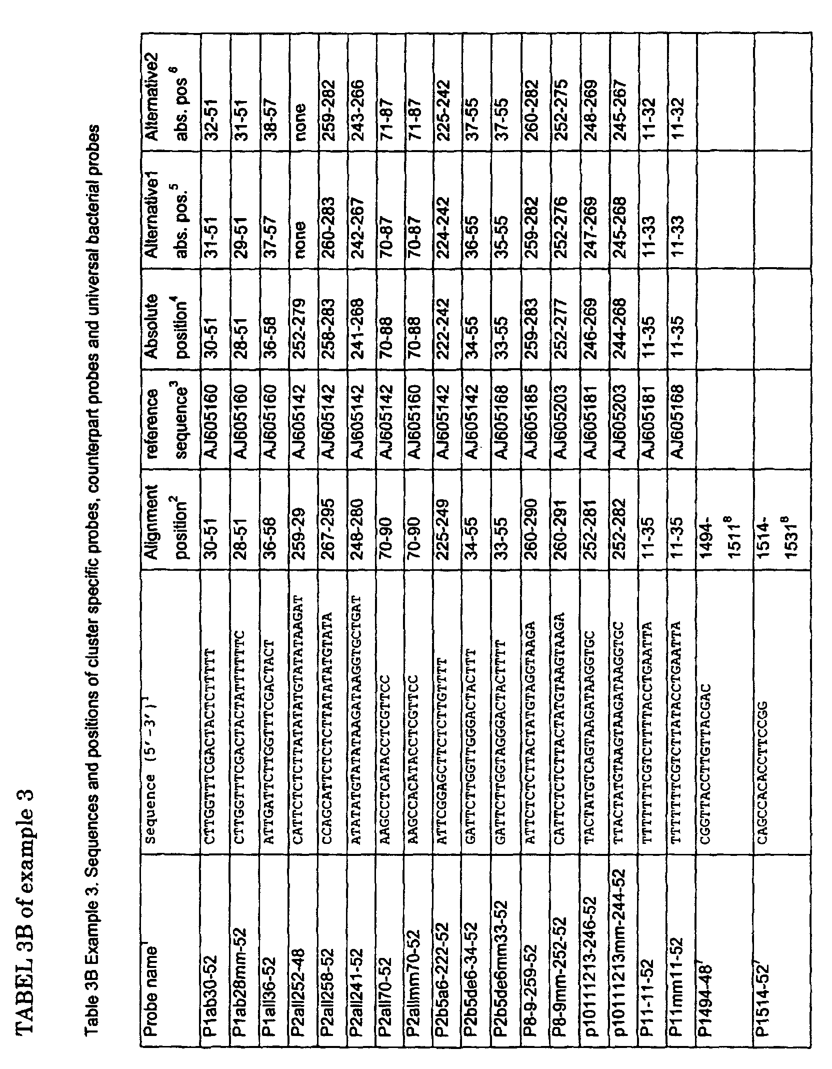

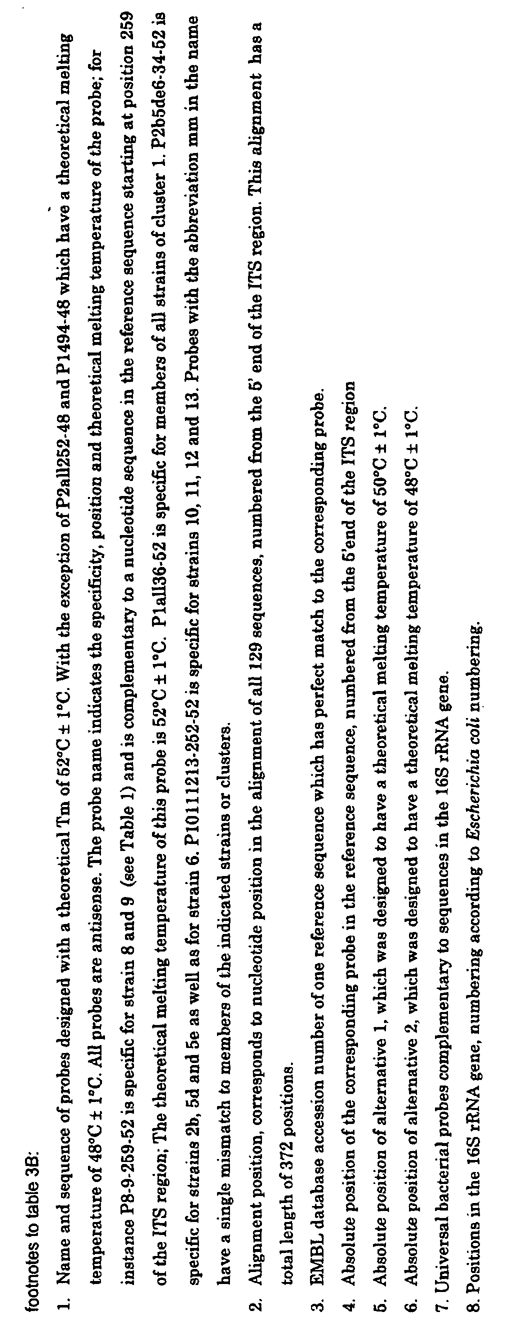

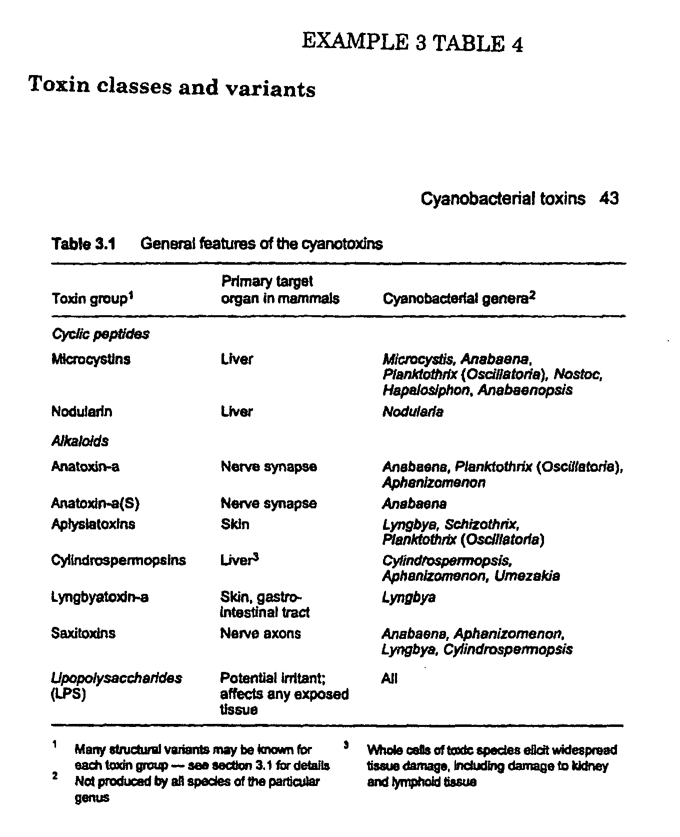

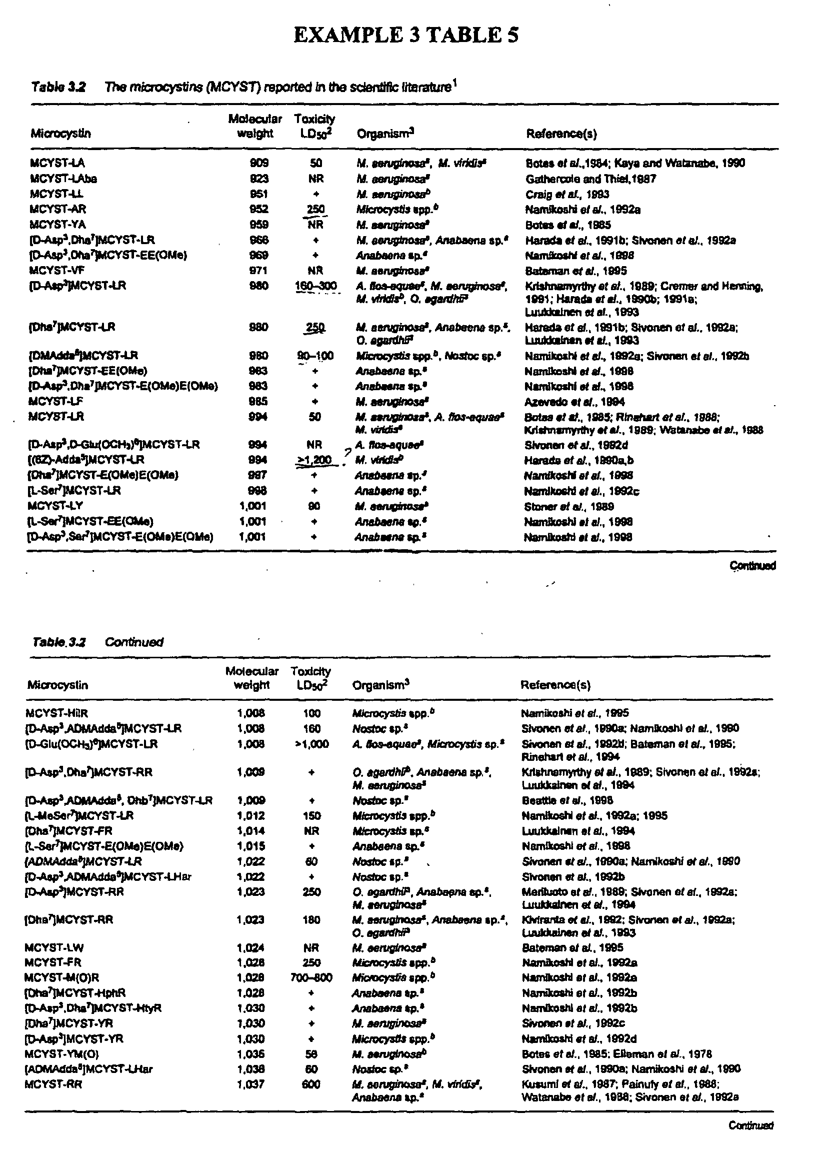

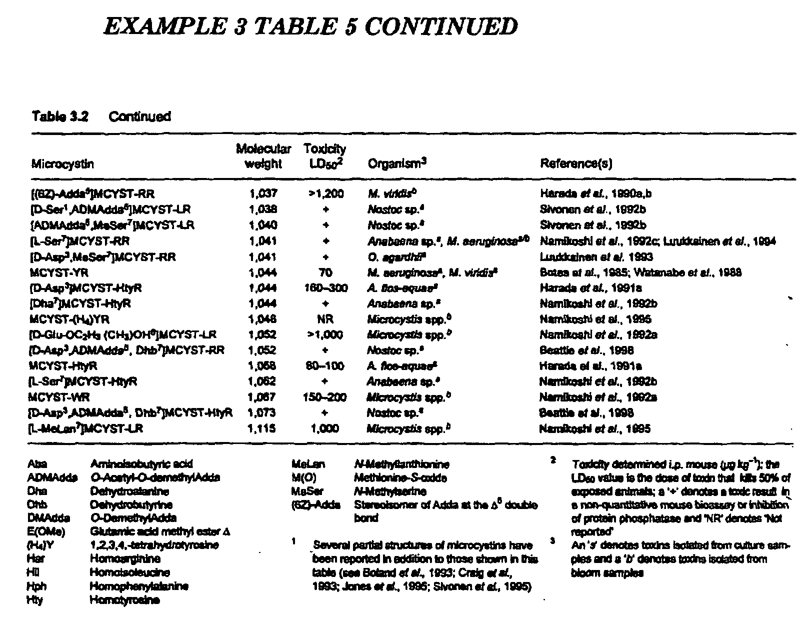

## Claims

1. A method comprising hybridising a probe from an rRNA-internal transcribed spacer region (ITS) located between sequences from a 16S rRNA gene and a 23S rRNA gene, of a reference cyanobacterium strain, with a preparation comprising nucleic acid from said region derived from a test cyanobacterium and determining whether hybridisation occurs.

2. A method according to claim 1, further comprising comparing the hybridisation result with a control hybridisation.

3. A method according to claim 1 or claim 2, further comprising classifying said test cyanobacterium on the basis of said hybridisation.

4. A method according to claim 3, wherein said classification comprising classifying said test cyanobacterium as a toxin producing strain.

5. A method according to claim 3 or claim 4, wherein said classification further comprises classifying said cyanobacterium as a surface layer forming strain.

6. A method according to any one of claims 3-5, further comprising classifying the test cyanobacterium as a high toxin producing strain.

7. A method according to any one of claims 1-6, wherein said hybridisation comprises hybridising said preparation with a panel of nucleic acid probes wherein said panel comprises said probe and at least one probe derived from the ITS of another reference cyanobacterium strain.

8. A method according to claim 7, wherein said panel comprises at least five probes, wherein each of said five probes is derived from ITS sequences of different reference cyanobacterium strains.

9. A method according to claim 7 or claim 8, wherein at least one of said probes comprises no mismatches with the sequence of the reference strain the probe is derived from, and at least one mismatch, and preferably at least two mismatches with the sequence of said region in at least one other reference cyanobacterium strain.

10. A method according to claim 9, wherein at least five of said probes comprise no mismatches with the sequence of said region in the reference strains they are derived from, and wherein each of said at least five probes comprises at least two mismatches with the sequence of said region in the other strains of said at least five reference strains.

11. A method according to any one of claims 1-10, wherein at least one of said probes comprises between 12 and 30 nucleotides.

12. A method according to claim 11, wherein said between 12 and 30 nucleotides are arranged consecutively in said region.

13. A method according to claim 11 or claim 12, wherein said probe comprises further nucleotides that are not derived from said region.

14. A method according to any one of claims 1-13, wherein at least one of said probes comprises a nucleic acid sequence as depicted in table 3A of example 3 or a functional equivalent thereof.

15. A method according to any one of claims 7-14, wherein at least two of said probes comprise a nucleic acid sequence as depicted in table 3A of example 3 or a functional equivalent thereof.

16. A method according to any one of claims 8-15, wherein at least five of said probes comprise a nucleic acid sequence as depicted in table 3A of example 3 or a functional equivalent thereof.

17. A method according to any one of claims 3-16, further comprising hybridising a control probe from said rRNA-internal transcribed spacer region (ITS) located between sequences from a 16S rRNA gene and a 23S rRNA gene, with a preparation comprising nucleic acid from said region derived from a test cyanobacterium and determining whether hybridisation occurs, wherein said control probe comprises sequence divergence with some strains of cyanobacteria, but not with other strains of cyanobacteria.

18. A method according to claim 17, wherein said control probe is selective for a cluster of cyanobacteria.

19. A method according to claim 18, wherein said control probe is selective for a cluster of cyanobacteria that comprises the strain that said probe is selective for, preferably said control probe comprises a sequence as depicted in table 3B of example 3.

20. A method according to any one of claims 1-19, wherein said preparation of nucleic acid of said test cyanobacterium is at least in part prepared through amplification of a sample comprising nucleic acid of said test cyanobacterium.

21. A method according to claim 20, wherein said amplification was performed using a cyanobacterium specific primer within the 16S, 23S or 16S-23S rRNA internal transcribed spacer gene sequence.

22. A method according to claim 21, wherein said primer comprises a sequence as depicted in table 1 of example 3.

23. A nucleic acid comprising a nucleic acid sequence as depicted in table 1 of example 3, or a nucleic acid sequence according to table 3 of example 3 or a functional equivalent thereof.

24. A panel of nucleic acids wherein at least one of said nucleic acids comprise a sequence as depicted in table 3A of example 3 or a functional equivalent thereof.

25. A panel of nucleic acids wherein at least five of said nucleic acids comprise a sequence as depicted in table 3A of example 3 or a functional equivalent thereof.

26. A nucleic acid array comprising a nucleic acid according to claim 23, or a panel of nucleic acids according to claim 24 or claim 25.

27. A chip comprising a nucleic acid according to claim 23 or an array according to claim 26.

28. An apparatus for analysing hybridisation signals comprising a panel of nucleic acids according to any one of claims 24-26 and/or a chip according to claim 27.

29. Use of a nucleic acid according to claim 23, a panel according to claim 24 or claim 25, a chip according to claim 27, or an apparatus according to claim 28, for classifying a cyanobacterium.

30. Use of a nucleic acid according to claim 23, a panel according to claim 241 or claim 25, a chip according to claim 27, or an apparatus according to claim 28, for classifying a cyanobacterium as a toxic or non-toxic cyanobacterium strain.

31. A method for typing a cyanobacterium isolate in a sample comprising subjecting said ITS sequences from said isolate to a denaturing gradient gel electrophoresis step and typing the strain from said step.

32. A method according to claim 31, wherein said typing comprises comparing the result of said step for said isolate with a reference strain.

33. A method according to claim 31 or 32; wherein said ITS sequences are provided through amplification of the ITS region of said cyanobacterium isolate.

34. A method according to claim 33, wherein said ITS sequences are amplified by using a cyanobacterium specific primer within the 16S, 23S or 16S-23S rRNA internal transcribed spacer gene sequence.

35. A method according to claim 34, wherein said cyanobacterium specific primer comprises a sequence as depicted in table 1 of example 3.
